(19) **Europäisches Patentamt**

**European Patent Office**

**Office européen des brevets**

(11) **EP 0 831 738 B1**

(12) **EUROPEAN PATENT SPECIFICATION**

(45) Date of publication and mention
of the grant of the patent:
**28.04.2004 Bulletin 2004/18**

(51) Int Cl.⁷: **A61B 5/00**

(86) International application number:
**PCT/US1996/008505**

(21) Application number: **96917037.2**

(22) Date of filing: **04.06.1996**

(87) International publication number:
**WO 1996/039926 (19.12.1996 Gazette 1996/55)**

(54) **ACTIVE PULSE BLOOD CONSTITUENT MONITORING**

ANALYSE VON BLUTBESTANDTEILEN GESTEUERT DURCH AKTIVE IMPULSE

CONTROLE DE CONSTITUANTS DU SANG PAR IMPULSIONS ACTIVES

(84) Designated Contracting States:
**AT BE CH DE DK ES FI FR GB GR IE IT LI LU MC NL PT SE**

(30) Priority: **07.06.1995 US 482071**

(43) Date of publication of application:
**01.04.1998 Bulletin 1998/14**

(73) Proprietor: **Masimo Corporation Irvine, CA 92614 (US)**

(72) Inventors:
• **KIANI-AZARBAYJANY, Esmaiel**
**Laguna Niguel, CA 92677 (US)**
• **DIAB, Mohamed, Kheir**
**Mission Viejo, CA 92691 (US)**
• **LEPPER, James, M., Jr.**
**Glendale, CA 91206-2808 (US)**

(74) Representative: **VOSSIUS & PARTNER**
**Postfach 86 07 67**
**81634 München (DE)**

(56) References cited:
**WO-A-90/04353**     **WO-A-92/17765**
**US-A- 4 883 055**     **US-A- 4 927 264**

EP 0 831 738 B1

**Description**

Background of the Invention

Field of the Invention

The present invention relates to noninvasive systems for monitoring blood glucose and other difficult to detect blood constituent concentrations, such as therapeutic drugs, drugs of abuse, carboxyhemoglobin, Methemoglobin, cholesterol.

Description of the Related Art

In the past, many systems have been developed for monitoring blood characteristics. For example, devices have been developed which are capable of determining such blood characteristics as blood oxygenation, glucose concentration, and other blood characteristics.

WO-A-90/04353 relates to a method for non-invasive intermittent and/or continuous hemoglobin, arterial oxygen content, and hematocrit determination. The method comprises measuring changes and mass of total hemoglobin and oxyhemoglobin and/or reduced hemoglobin and correlating these measured changes with known or measured changes in blood volume in a specific portion of an animal or human. The measuring of the changes in the mass of hemoglobin species may accomplished by subjecting a portion of a body to electromagnetic radiation and measurement of the attenuation of the radiation resulting from the absorption of said radiation by said body portion. The volume change may be a passive volume change or may be actively reduced by external means, e.g., by a mechanically limited and known change in length of a volume of known cross-sectional area.

US-A-4 883 055 relates to an artificially induced blood pulse used for measuring oxygen saturation in an arterial blood which is produced by a cuff, wrapped around a body member having an artery upstream from a testing side, when a squeezing pulse is applied by the cuff to the body member. Electrical stimulation may be used to contract muscles for artificially inducing blood pulses.

However, significant difficulties have been encountered when attempting to determine blood glucose concentration accurately using noninvasive blood monitoring systems such as by means of spectroscopic measurement.

The difficulty in determining blood glucose concentration accurately may be attributed to several causes. One of the significant causes is that blood glucose is typically found in very low concentrations within the bloodstream (e. g., on the order of 100 to 1,000 times lower than hemoglobin) so that such low concentrations are difficult to detect noninvasively, and require a very high signal-to-noise ratio. Additionally, with spectroscopic methods, the optical characteristics of glucose are very similar to those of water which is found in a very high concentration within the blood. Thus, where optical monitoring systems are used, the optical characteristics of water tend to obscure the characteristics of optical signals due to glucose within the bloodstream. Furthermore, since each individual has tissue, bone and unique blood properties, each measurement typically requires calibration for the particular individual.

In an attempt to accurately measure blood glucose levels within the bloodstream, several methods have been used. For example, one method involves drawing blood from the patient and separating the glucose from the other constituents within the blood. Although fairly accurate, this method requires drawing the patient's blood, which is less desirable than noninvasive techniques, especially for patients such as small children or anemic patients. Furthermore, when blood glucose monitoring is used to control the blood glucose level, blood must be drawn three to six times per day, which may be both physically and psychologically traumatic for a patient. Other methods contemplate determining blood glucose concentration by means of urinalysis or some other method which involves pumping or diffusing body fluid from the body through vessel walls or using other body fluids such as tears or sweat. However, such an analysis tends to be less accurate than a direct measurement of glucose within the blood, since the urine, or other body fluid, has passed through the kidneys (or skin in the case of sweat). This problem is especially pronounced in diabetics. Furthermore, acquiring urine and other body fluid samples is often inconvenient.

As is well known in the art, different molecules, typically referred to as constituents, contained within the medium have different optical characteristics so that they are more or less absorbent at different wavelengths of fight. Thus, by analyzing the characteristics of the fleshy medium containing blood at different wavelengths, an indication of the composition of the blood in the fleshy medium may be determined.

Spectroscopic analysis is based in part upon the Beer-Lamhert law of optical characteristics for different elements. Briefly, Beer-Lambert's law states that the optical intensity of light through any medium comprising a single substance is proportional to the exponent of the product of path length through the medium times the concentration of the substance within the medium times the extinction coefficient of the substance. That is,

$$I = I_o e^{-(pl \cdot c \cdot \varepsilon)} \tag{1}$$

where pl represents the path length through the medium, c represents the concentration of the substance within the medium, $\varepsilon$ represents the absorption (extinction) coefficient of the substance and $I_o$ is the initial intensity of the light from the light source. For optical media which have several constituents, the optical intensity of the light received from the illuminated medium is proportional to the exponent of the path length through the medium times the concentration of the first substance times the optical absorption coefficient associated with the first substance, plus the path length times the concentration of the second substance times the optical absorption coefficient associated with the second substance, etc. That is,

$$I = I_o e^{-(pl \cdot c_1 \cdot \varepsilon_1 + pl \cdot c_2 \cdot \varepsilon_2 + etc.)} \tag{2}$$

where $\varepsilon_n$ represents the optical absorption (extinction) coefficient of the $n^{th}$ constituent and $c_n$ represents the concentration of the $n^{th}$ constituent.

Summary of the Invention

The system according to the present invention is defined by the features of the claims.

Due to the parameters required by the Beer-Lambert law, the difficulties in detecting glucose concentration arise from the difficulty in determining the exact path length through a medium (resulting from transforming the multipath signal to an equivalent single-path signal), as well as difficulties encountered due to low signal strength resultant from a low concentration of blood glucose. Path length through a medium such as a fingertip or earlobe is very difficult to determine, because not only are optical wavelengths absorbed differently by the fleshy medium, but also the signals are scattered within the medium and transmitted through different paths. Furthermore, as indicated by the above equation (2), the measured signal intensity at a given wavelength does not vary linearly with respect to the path length. Therefore, variations in path length of multiple paths of light through the medium do not result in a linear averaging of the multiple path lengths. Thus, it is often very difficult to determine an exact path length through a fingertip or earlobe for each wavelength.

In conventional spectroscopic blood constituent measurements, such a blood oxygen saturation, light is transmitted at various wavelengths through the fleshy medium. The fleshy medium (containing blood) attenuates the incident light and the detected signal can be used to calculate certain saturation values. In conventional spectroscopic blood constituent measurements, the heart beat provides a minimal modulation to the detected attenuated signal in order to allow a computation based upon the AC portion of the detected signal with respect to the DC portion of the detected signal, as disclosed in U.S. Patent No. 4,407,290. This AC/DC operation normalizes the signal and accounts for variations in the pathlengths, as well understood in the art.

However, the natural heart beat generally provides approximately a 1-10% modulation (AC portion of the total signal) of the detected signal when light is transmitted through a patient's digit or the like. That is, the variation in attenuation of the signal due to blood may be only 1% of the total attenuation (other attenuation being due to muscle, bone, flesh, etc.). In fact, diabetes patients typically have even lower modulation (e.g., .01 - .1%). Therefore, the attenuation variation (AC portion of the total attenuation) due to natural pulse can be extremely small. In addition, the portion of the pulse modulation which is due to glucose is roughly only 9% of the pulse (approximately 1/11) at a wavelength of 1330-1340 nm where glucose absorbs effectively. Furthermore, to resolve glucose from 5 mg/dl to 1005 mg/dl in increments or steps of 5 mg/dl, requires resolution of 1/200 of the 9% of the modulation which is due to glucose. Accordingly, by way of three different examples -- one for a healthy individual, one for a diabetic with a strong pulse, and one for a diabetic with a weak pulse -- for absorption at 1330 nm, the system would require resolution as follows.

Example 1: Healthy individuals where natural pulse provides attenuation modulation of 1% at 1330 nm:

    a. Natural modulation due to pulse is approximately 1% (1/100).
    b. Portion of natural modulation due to glucose is approximately 9% (1/11).
    c. To resolve glucose from 5 - 1005 mg/dl requires resolution of 1/200 (i.e., there are 200, 5 mg/dl steps between 5 and 1005 mg/dl).
    Required Total Resolution is product of a-c:

$$1/100 * 1/11 * 1/200 = 1/220,000$$

Example 2: Diabetic where natural pulse provides attenuation modulation of .1% at 1330 nm

a. Natural modulation due to pulse approximately .1% (1/1000).
b. Portion of natural modulation due to glucose is approximately 9% (1/11)
c. To resolve glucose from 5 - 1005 mg/dl requires resolution of 11200.
Required total resolution is product of a-c:

$$1/1000 * 1/11 * 1/200 = 1/2,200,000$$

Example 3: Diabetic where natural pulse provides attenuation modulation of .01%

a. Natural modulation due to pulse approximately .01% (1/10,000).
b. Portion of natural modulation due to glucose is approximately 9% (1/11).
c. To resolve glucose from 5 - 1005 mg/dl requires resolution of 1/200. Required total resolution is product of a-c:

$$1/10,000 * 1/11 * 1/200 = 1/22,000,000$$

As seen from the above three examples which provide the range of modulation typically expected among human patients, the total resolution requirements range from 1 in 220,000 to 1 in 22,000,000 in order to detect the attenuation which is due to glucose based on the natural pulse for the three examples. This is such a small portion that accurate measurement is very difficult. In most cases, the noise accounts for a greater portion of the AC portion (natural modulation due to pulse) of the signal than the glucose, leaving glucose undetectable. Even with state of the art noise reduction processing as described in U.S. Patent No. 5,482,036 signals may be resolved to a level of approximately 1/250,000. This is for an 18-bit system. With a 16-bit system, resolution is approximately 1/65,000. In addition, LEDs are often noisy such that even if resolution in the system is available to 1/250,000, the noise from the LEDs leave glucose undetectable.

To overcome these obstacles, it has been determined that by actively inducing a change in the flow of blood in the medium under test such that the blood flow varies in a controlled manner periodically, modulation can be obtained such that the portion of the attenuated signal due to blood becomes a greater portion of the total signal than with modulation due to the natural pulse. This leads to the portion of total attenuation due to glucose in the blood being a greater portion of the total signal. In addition, the signal can be normalized to account for factors such as source brightness, detector responsiveness, tissue or bone variation. Changes in blood flow can be induced in several ways, such as physically perturbing the medium under test or changing the temperature of the medium under test. In the present embodiment, by actively inducing a pulse, a 10% modulation in attenuation (1/10 of the total attenuation) is obtained, regardless of the patient's natural pulse modulation (whether or not the patient is diabetic). Accordingly, at 1330 nm with actively induced changes in blood flow, the resolution required is 1/10 * 1/11 * 1/200 or 1/22,000 (where 1/10 is the active pulse attenuation modulation (the modulation obtained by induced blood flow changes), 1/11 is the portion of the modulation due to glucose, and 1/200 the resolution required to obtain glucose in 5mg/dl increments from 5 - 1005 mg/dl). As will be understood from the discussion above, such resolution can be obtained, even in a 16 bit system. In addition, the resolution is obtainable beyond the noise floor, as described herein.

In conventional blood constituent measurement through spectroscopy, perturbation of the medium under test has been avoided because oxygen (the most commonly desired parameter) is not evenly dispersed in the arterial and venous blood. Therefore, perturbation obscures the ability to determine the arterial oxygen saturation because that venous and arterial blood become intermingled. However, glucose is evenly dispersed in blood fluids, so the mixing of venous and arterial blood and interstitial fluids should have no significant effect on the glucose measurements. It should be appreciated that this technique will be effective for any substance evenly dispersed in the body fluids (e.g., blood, interstitial fluids, etc.).

One aspect of the present invention involves a system for non-invasively monitoring a blood constituent concentration in a living subject. The system comprises a light source which emits radiation at a plurality of wavelengths and an active pulse inducement device which, independent of the natural flow of blood in the fleshy medium, causes a periodic change in the volume of blood in the fleshy medium. An optical detector positioned to detect light which has propagated through the fleshy medium is configured to generate an output signal indicative of the intensity of the radiation after attenuation through the fleshy medium. A signal processor responds to the output signal to analyze the output signal to extract portions of the signal due to optical characteristics of the blood to determine the concentration of the constituent within the subject's bloodstream.

In one embodiment, the system further comprises a receptacle which receives the fleshy medium, the receptacle further having an inflatable bladder.

In one embodiment, the system has a temperature variation element in the receptacle, the temperature variation element varies (e.g., increases) the temperature of the fleshy medium in order to induce a change (e.g., increase) in the flow of blood in the fleshy medium.

Another aspect of the present invention involves a system for non-invasively monitoring blood glucose concentration within a patient's bloodstream. A light source emits optical radiation at a plurality of frequencies, and a sensor receives a fleshy medium of the patient, the fleshy medium having flowing blood. A fluid (e.g., blood and interstitial fluids) volume change inducement device causes a cyclic change in the volume of blood in the fleshy medium. An optical detector positioned to receive the optical radiation after transmission through a portion of the fleshy medium responds to the detection of the optical radiation to generate an output signal indicative of the intensity of the optical radiation. A signal processor coupled to the detector receives the output signal, and responds to the output signal to generate a value representative of the glucose concentration in the blood of the patient.

Yet another aspect of the present invention involves a method of non-invasively determining a concentration of a blood constituent. The method comprises a plurality of steps. Optical radiation is transmitted through a medium having flowing fluid, wherein the fluid has a concentration of the fluid constituent. A periodic change in the volume of the fluid in the medium is actively induced. The optical radiation after transmission through at least a portion of the medium is detected and a signal indicative of the optical characteristics of the medium is generated. The signal is analyzed to determine the concentration of the blood constituent. In one embodiment, the fluid constituent comprises blood glucose.

A further aspect of the present invention involves a method of actively varying the attenuation of optical radiation due to blood in a fleshy medium. The method comprises a plurality of steps. Optical radiation is transmitted through the fleshy medium. A periodic change in the volume of blood is actively influenced in the medium. The optical radiation is detected after attenuation through the fleshy medium and an output signal indicative of the intensity of the attenuated signal is generated.

Brief Description of the Drawings

Figure 1 depicts an embodiment of a blood glucose monitor of the present invention.
Figure 2 depicts an example of a physiological monitor in accordance with the teachings of the present invention.
Figure 2A illustrates an example of a low noise emitter current driver with accompanying digital to analog converter.
Figure 2B depicts an embodiment of Figure 2 with added function for normalizing instabilities in emitters of Figure 2.
Figure 2C illustrates a comparison between instabilities in selected emitters.
Figure 3 illustrates the front end analog signal conditioning circuitry and the analog to digital conversion circuitry of the physiological monitor of Figure 2.
Figure 4 illustrates further detail of the digital signal processing circuitry of Figure 2.
Figure 5 illustrates additional detail of the operations performed by the digital signal processing circuitry of Figure 2.
Figure 6 illustrates additional detail regarding the demodulation module of Figure 5.
Figure 7 illustrates additional detail regarding the decimation module of Figure 5.
Figure 8 represents a more detailed block diagram of the operations of the glucose calculation module of Figure 5.
Figure 9 illustrates the extinction coefficient versus wavelength for several blood constituents.
Figure 10 - 12 depict one embodiment of a probe which can be used to induce an active pulse in accordance with the principals of the present invention.
Figure 13 depicts an example of an active pulse signal where the modulation is 10% of the entire attenuation through the finger.

Detailed Description of the Invention

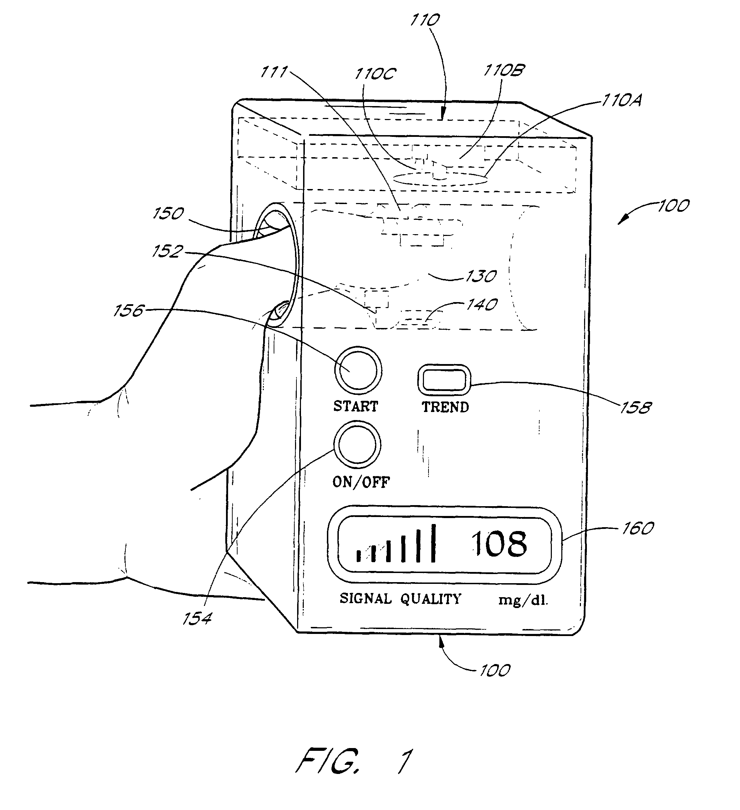

Figure 1 depicts one embodiment of a blood glucose monitor system 100 in accordance with the teachings of the present invention. The glucose monitor 100 of Figure 1 has an emitter 110 such as light emitting diodes or a light with a filter wheel.

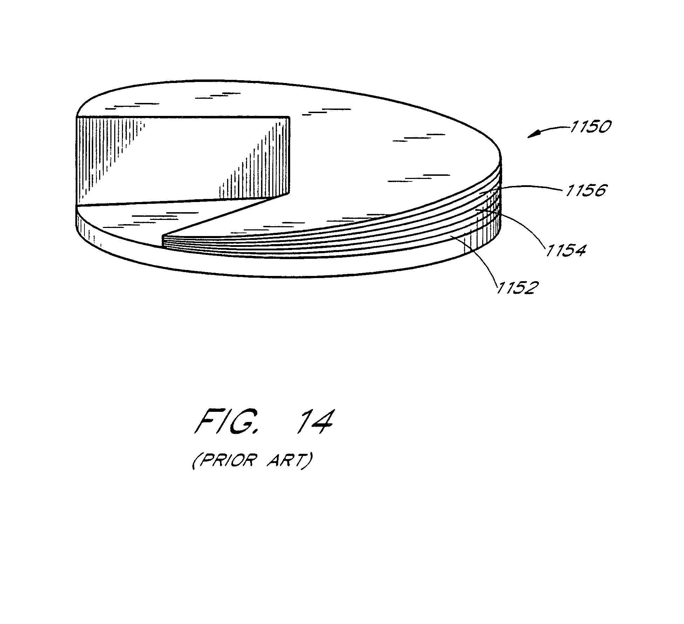

The filter wheel with a broadband light is depicted in Figure 1. This arrangement comprises a filter wheel 110A, a motor 110B, and a broadband light source 110C. Advantageously, this unit can be made relatively inexpensively as a replaceable unit. The filter wheel is advantageously a conventional dichroic filter or filter made in accordance with the discussion of Figures 14-21.

The monitor system 100 has a detector 140, such as a photodetector. The blood glucose monitor 100 also has a pressure inducing cuff 150 to physically squeeze a digit 130 in order to periodically induce a "pulse" in the fluid (i.e., actively vary the flow of fluid) in a digit 130. In other words, a device influences a change in the volume of blood in the digit or other fleshy medium. A window 111 is positioned to allow light from the emitter 110 to pass through the window 11 and transmit through the digit 130. This intentional active perturbation of the blood in the digit or medium under test is further referred to herein as an "active pulse." The blood glucose monitor also has a display 160 which may be used to indicate such parameters as glucose concentration and signal quality. Advantageously, the blood glucose monitor also has a power switch 154, a start switch 156 and a trend data switch 158.

Other methods of inducing a pulse are also possible. For instance, the fleshy medium under test, such as the patient's digit, could be perturbed with a pressure device 152 (depicted in dotted lines in Figure 1). Other methods of inducing a pulse could be utilized such as temperature fluctuations or other physiological changes which resuh in a fluctuation (modulation) of blood volume through the fleshy medium. All external methods (as opposed to the natural heart beat) that actively vary the blood volume in the medium under test are collectively referred to herein as inducing an "active pulse." In the present embodiment, 10% modulation in the total attenuation is obtained through the active induction of a pulse. The 10% modulation is selected as a level of minimal perturbation to the system. Too much perturbation of the medium will change the optical characteristics of the medium under test. For instance, with substantial modulation (e.g., 40 - 50%), the perturbation could impact scattering within the medium under test differently for different wavelengths, thus causing inaccurate measurements.

The pressure device 152, the cuff 150 and the use of temperature to induce a pulse in the fleshy medium are advantageous in that they can be used with minimal or no movement of the flashy medium in the area through which light is transmitted. This is possible through inducing the pulse at a location proximal or distal from the area receiving the incident light. The advantage of minimal movement is that movement in the area of the fleshy medium under test causes variation in the detected signal other than due to the varying fluid volume (e.g., blood and interstitial fluid) flow. For instance, physical perturbation in the area of light transmission can cause changes in the fight coupling to the medium under test resulting in variations in attenuation which are not due to changes in fluid volume in the area of light transmission. These other variations comprise additional noise that should be removed for accurate measurement.

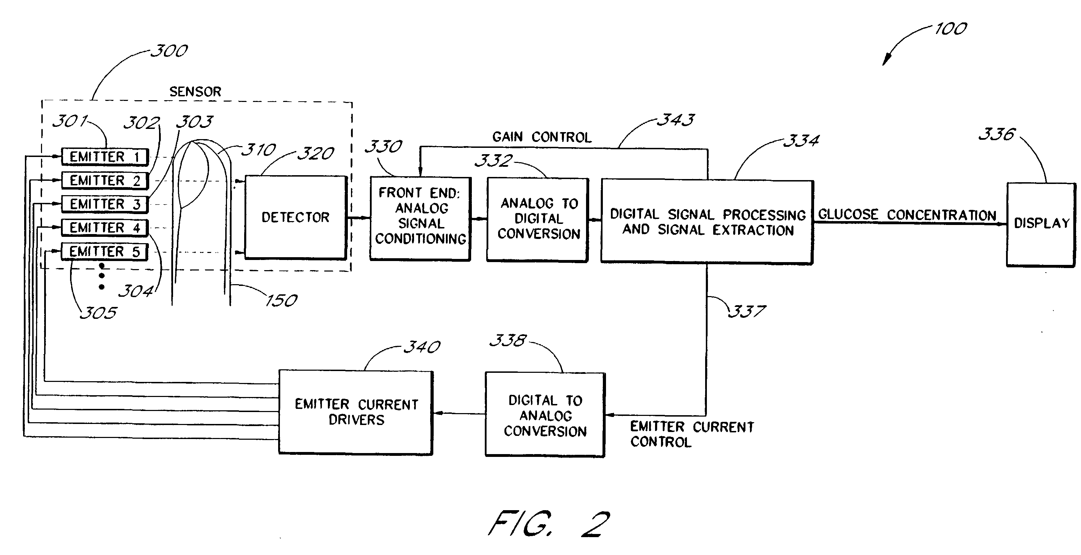

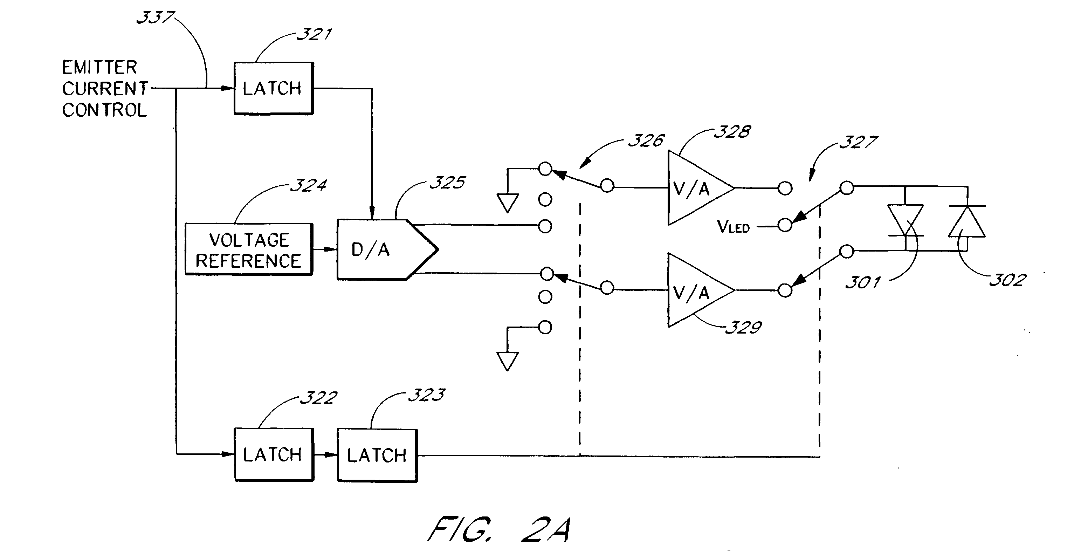

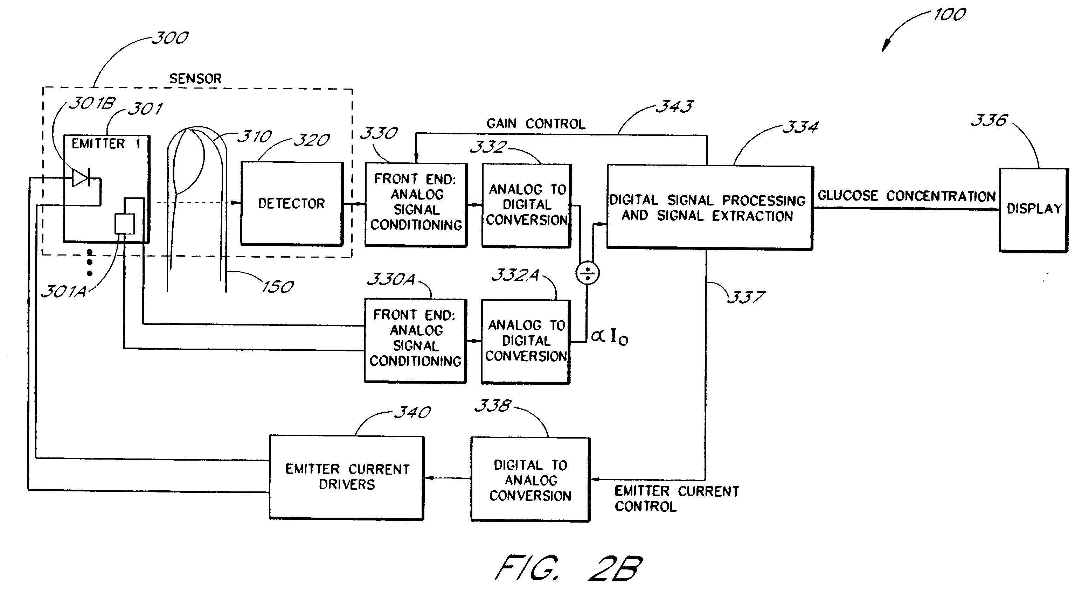

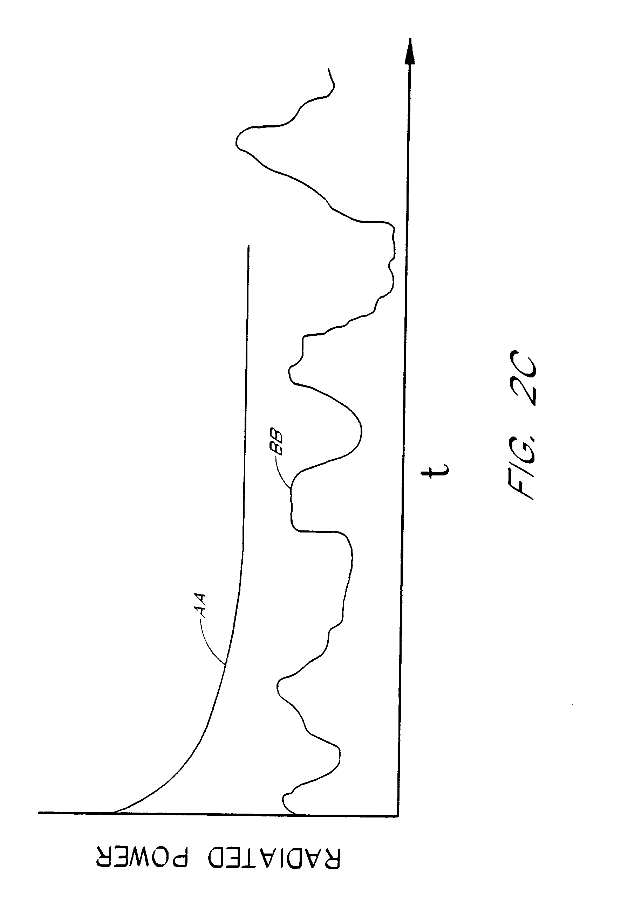

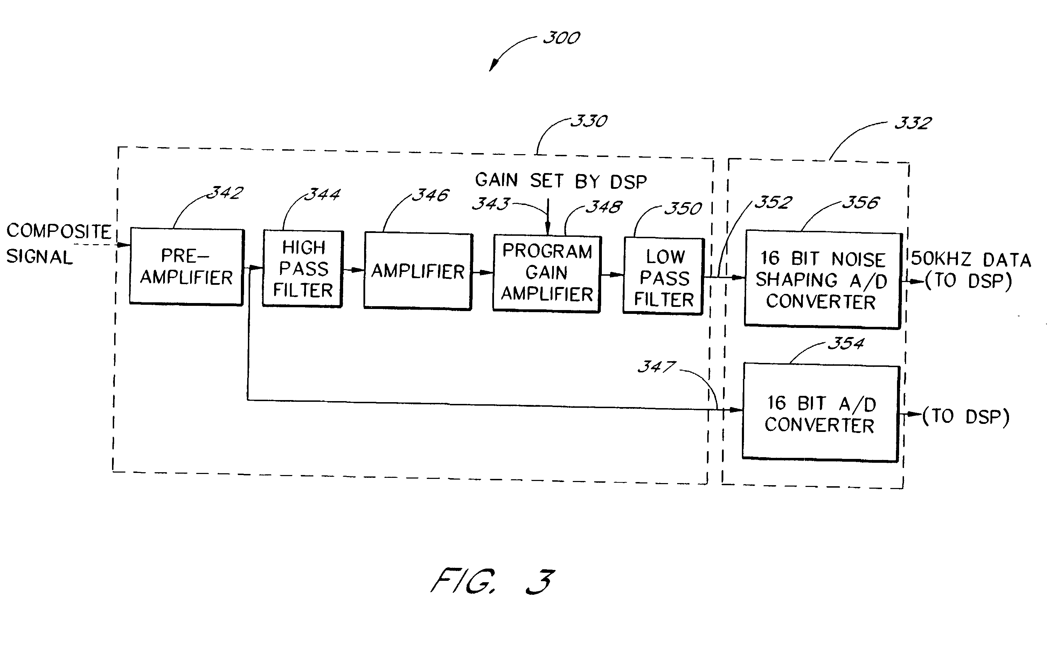

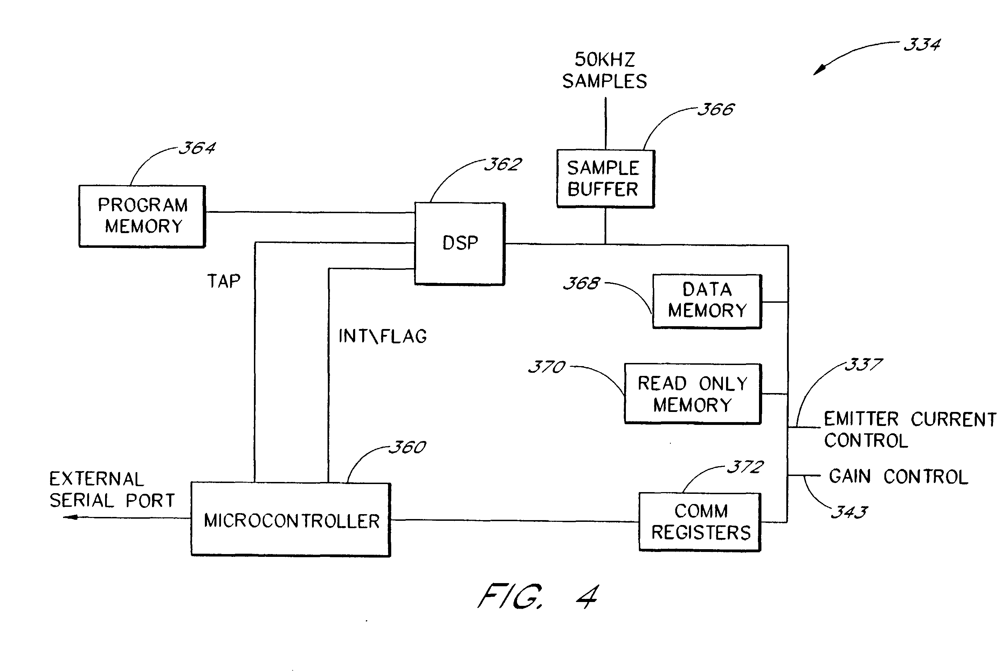

Figure 2-4 depict a schematic block diagram of the blood glucose monitoring system 100 in accordance with the teachings of the present invention. Figure 2 illustrates a general hardware block diagram. A sensor 300 has multiple light emitters 301 - 305 such as LED's. In the present embodiment, each LED 301-305 emits light at a different wavelength.

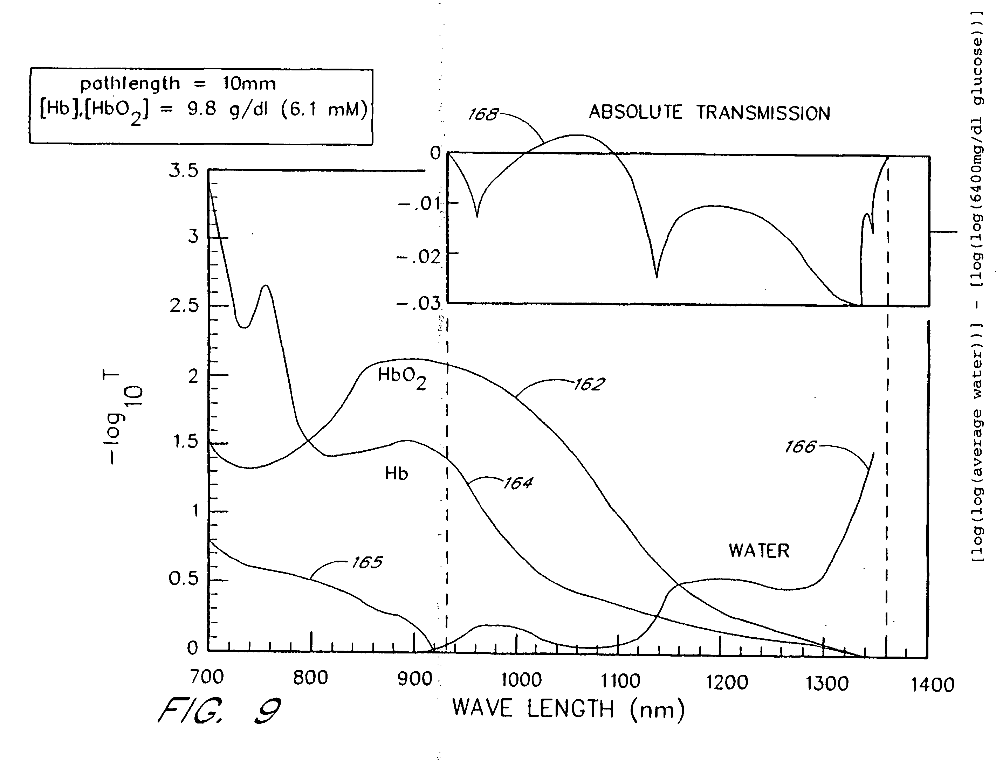

As well understood in the art, because Beer-Lambert's law contains a term for each constituent which attenuates the signal, one wavelength is provided for each constituent which is accounted for. For increased precision, the wavelengths are chosen at points where attenuation for each particular constituent is the greatest and attenuation by other constituents is less significant. Figure 9 depicts the extinction coefficient on a log scale vs. wavelength for principal blood constituents. The curve 162 represents the extinction coefficient for oxyhemoglobin; the curve 164 represents the extinction coefficient for hemoglobin; the curve 165 represents the extinction coefficient for carboxyhemoglobin; and the curve 166 represents the extinction coefficient for water. Depicted on the same horizontal axis with a different vertical axis is a curve 16B which represents the extinction coefficient for glucose in body fluids. It should be noted that the curve 168 is placed above the other curves and is greatly amplified, and therefore is not to scale on the graph. If the glucose curve were graphed on the same scale as the other constituents, it would simply appear as flat line at '0' on the vertical axis in the wavelength range from 900 - 1400 mm. The provision for a separate vertical axis provides for amplification in order to illustrate at which wavelengths glucose attenuates the most in the range of interest. The vertical axis for the glucose curve 168 also represents a different value. In Figure 9, the vertical axis for the curve 168 is in terms of the absolute transmission on the following log scale:

$$[\log(\log(\text{average water}))]\text{-}[\log(\log(6400 \text{ mg/dl glucose}))]$$

However, for purposes of choosing appropriate wavelengths, the scale is of less significance than the points at which Glucose and the other constituents show good attenuation and the attenuation is not totally obscured by other constituents in the medium.

In the present embodiment, advantageous wavelengths for the emitters 301-305 (or to obtain with the filter wheel and signal processing) are 660 nm (good attenuation hemoglobin), 905 nm (good attenuation from oxyhemoglobin), 1270 nm (good attenuation by water, and little attenuation by other constituents) 1330-1340 nm (good attenuation due to Glucose in the area of the graph labelled A of Figure 9, not totally obscured by the attenuation due to water), and 1050 nm (an additional point for good attenuation from Glucose). The use of two wavelengths to account for glucose attenuation provides overspecification of the equations. Overspecification of the equations discussed below increases resolution. Additional wavelengths to account for other constituents such as fats and proteins or others could also be included. For instance, an additional wavelength at 1100 nm could be added (good attenuation from proteins) and 920 nm (good attenuation from fats). Another constituent often of interest is carboxyhemoglobin. A wavelength for carboxyhemoglobin is advantageously selected at 700-730 nm.

In addition to using multiple precise LEDs, an optical spectroscopic system for generating the optical characteristics over many wavelengths can be used.

The sensor 300 further comprises a detector 320 (e.g., a photodetector), which produces an electrical signal corresponding to the attenuated light energy signals. The detector 320 is located so as to receive the light from the emitters 301-305 after it has propagated through at least a portion of the medium under test. In the embodiment depicted in Figure 2, the detector 320 is located opposite the LED's 301 - 305. The detector 320 is coupled to front end analog signal conditioning circuity 330.

The front end analog signal conditioning circuitry 330 has outputs coupled to analog to digital conversion circuit 332. The analog to digital conversion circuitry 332 has outputs coupled to a digital signal processing system 334. The digital signal processing system 334 provides the desired parameter as an output for a display 336. The display 336 provides a reading of the blood glucose concentration.

The signal processing system also provides an emitter current control output 337 to a digital-to-analog converter circuit 338 which provides control information for emitter drivers 340. The emitter drivers 340 couple to the emitters 301-305. The digital signal processing system 334 also provides a gain control output 342 for the front end analog signal conditioning circuitry 330.

Figure 2A illustrates a preferred embodiment for the emitter drivers 340 and the digital to analog conversion circuit 338. The driver depicted in Figure 2a is depicted for two LEDs coupled back-to-back. However, additional LEDs (preferably coupled back-to-back to conserve connections) can be coupled to the D/A converter 325 through additional multiplexing circuitry (not shown). As depicted in Figure 2A, the driver comprises first and second input latches 321, 322, a synchronizing latch 323, a voltage reference 324, a digital to analog conversion circuit 325, first and second switch banks 326, 327, first and second voltage to current converters 328, 329 and the LED emitters 301, 302 corresponding to the LED emitters 301-302 of Figure 2.

The preferred driver depicted in Figure 2A is advantageous in that much of the noise in the blood glucose system 100 of Figure 2 is caused by the LED emitters 301-305. Therefore, the emitter driver circuit of Figure 2A is designed to minimize the noise from the emitters 301-305. The first and second input latches 321, 324 are connected directly to the DSP bus. Therefore, these latches significantly minimize the bandwidth (resulting in noise) present on the DSP bus which passes through to the driver circuitry of Figure 2A. The output of the first and second input latches only changes when these latches detect their address on the DSP bus. The first input latch receives the setting for the digital to analog converter circuit 325. The second input latch receives switching control data for the switch banks 326, 327. The synchronizing latch accepts the synchronizing pulses which maintain synchronization between the activation of emitters 301, 302 (and the other emitters 303-305 not depicted in Figure 2a) and the analog to digital conversion circuit 332.

The voltage reference is also chosen as a low noise DC voltage reference for the digital to analog conversion circuit 325. In addition, in the present embodiment, the voltage reference has an lowpass output filter with a very low corner frequency (e.g., 1Hz in the present embodiment). The digital to analog converter 325 also has a lowpass filter at its output with a very low corner frequency (e.g., 1 Hz). The digital to analog converter provides signals for each of the emitters 301, 302 (and the remaining emitters 303-305, not depicted in Figure 2a).

In the present embodiment, the output of the voltage to current converters 328, 329 are switched such that with the emitters 301, 302 connected in back-to-back configuration, only one emitter is active an any given time. A refusal position for the switch 326 is also provided to allow the emitters 301 and 302 to both be off when one of the other emitters 303 - 305 is on with a similar switching circuit. In addition, the voltage to current converter for the inactive emitter is switched off at its input as well, such that it is completely deactivated. This reduces noise from the switching and voltage to current conversion circuitry. In the present embodiment, low noise voltage to current converters are selected (e.g., Op 27 Op Amps), and the feedback loop is configured to have a low pass filter to reduce noise. In the present embodiment, the low pass filtering function of the voltage to current converter 328, 329 has a corner frequency just above the switching speed for the emitters. Accordingly, the preferred driver circuit of Figure 2a, minimizes the noise of the emitters 301, 302.

As represented in Figure 2, the light emitters 301-305 each emits energy which is absorbed by the finger 310 and received by the detector 320. The detector 320 produces an electrical signal which corresponds to the intensity of the light energy striking the photodetector 320. The front end analog signal conditioning circuitry 330 receives the intensity signals and filters and conditions these signals as further described below for further processing. The resultant signals are provided to the analog-to-digital conversion circuitry 332 which converts the analog signals to digital signals for further processing by the digital signal processing system 334. The digital signal processing system 334 utilizes the signals in order to provide blood glucose concentration. In the present embodiment, the output of the digital signal processing system 334 provides a value for glucose saturation to the display 336. Advantageously, the signal processing system 334 also store data over a period of time in order to generate trend data and perform other analysis on the data over time.

The digital signal processing system 334 also provides control for driving the light emitters 301-305 with an emitter current control signal on the emitter current control output 337. This value is a digital value which is converted by the digital-to-analog conversion circuit 338 which provides a control signal to the emitter current drivers 340. The emitter current drivers 340 provide the appropriate current drive for the emitters 301-305.

In the present embodiment, the emitters 301 - 305 are driven via the emitter current driver 340 to provide light transmission with digital modulation at 625 Hz. In the present embodiment, the light emitters 301-305 are driven at a power level which provides an acceptable intensity for detection by the detector and for conditioning by the front end analog signal conditioning circuitry 330. Once this energy level is determined for a given patient by the digital signal processing system 334, the current level for the emitters is maintained constant. It should be understood, however, that the current could be adjusted for changes in the ambient room light and other changes which would effect the voltage input to the front end analog signal conditioning circuitry 330. In the present invention, light emitters are modulated as follows: for one complete 625 Hz cycle for the first wavelength, the first emitter 301 is activated for the first tenth of the cycle, and off for the remaining nine-tenths of the cycle; for one complete 625 Hz second wavelength cycle, the second fight emitter 302 is activated for the one tenth of the cycle and off for the remaining nine-tenths cycle; for one 625 Hz third wavelength cycle, the third light emitter 303 is activated for one tenth cycle and is off for the remaining nine-tenths cycle; for one 625 Hz fourth wavelength cycle, the fourth light emitter 304 is activated for one tenth cycle and is off for the remaining nine-tenths cycle; and for one 625 Hz fifth wavelength cycle, the fifth light emitter 305 is activated for one tenth cycle and is off for the remaining nine-tenths cycle. In order to receive only one signal at a time, the emitters are cycled on and off alternatively, in sequence, with each only active for a tenth cycle per 625 Hz cycle and a tenth cycle separating the active times.

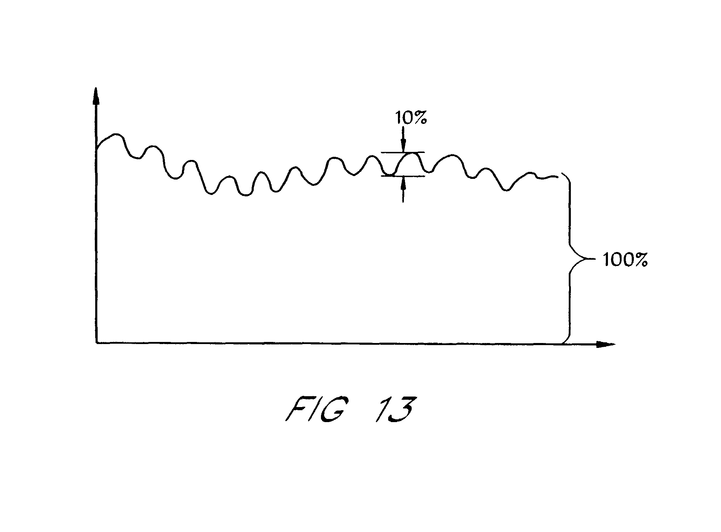

The light signal is attenuated (amplitude modulated) by the blood (with the volume of blood changing through cyclic active pulse in the present embodiment) through the finger 310 (or other sample medium). In the present embodiment, the fingertip 130 is physiologically altered on a periodic basis by the pressure device 150 (or the active pulse device) so that approximately 10% amplitude modulation is achieved. That is, enough pressure is applied to the fingertip 310 to evacuate a volume of body fluid such that the variation in the overall difference in optical attenuation observed between the finger tip 310 when full of blood and the finger tip 310 when blood is evacuated, is approximately 10%. For example, if the transmission of optical radiation through the fingertip 310 is approximately 0.4%, then the fingertip 310 would have to be physiologically altered to evacuate enough blood so that the attenuation of the fingertip having fluid evacuated would be on the order to 0.36%. Figure 13 depicts an example of an active pulse signal where the modulation is 10% of the entire attenuation through the finger. The 10% is obtained by varying the volume of blood enough to obtain the cyclic modulation depicted in Figure 13. As explained above, the 10% modulation is chosen as sufficient to obtain information regarding glucose concentrations, yet cause minimal perturbation to the system. Minimal perturbation is advantageous due to the optical variations caused by perturbing the system. The level of perturbation is advantageously below a level that causes significant variations in optical properties in the system, which variations affect different wavelengths differently.

In one advantageous embodiment, physiological altering of the fingertip 310 is accomplished by the application of periodic gentle pressure to the patient's finger 310 with the pressure cuff 150 (Figure 1). The finger 310 could also be perturbed by the pressure device 152 (Figure 1) or with temperature.

The modulation is performed at a selected rate. A narrow band pass filter may then be employed to isolate the frequency of interest. In the present embodiment, the modulation obtained through influencing an active pulse preferably occurs at a rate just above the normal heart rate (for instance, 4 Hz). In one embodiment, the system checks the heart rate and sets the active pulse rate such that it is above the natural heart rate, and also away from harmonics of the natural pulse rate. This allows for easy filtering with a very narrow band-pass fitter with a center frequency of at the selected active pulse rate (e.g., 4 Hz or the rate automatically selected by the system to be away from the fundamental natural heart rate frequency and any harmonics to the fundamental frequency). However, a frequency in or below the range of normal heart rate could also be used. Indeed, in one embodiment, the frequency tracks the heart rate, in which case the active pulse operates in conjunction with the natural pulse to increase the change in volume of flow with each heart beat.

The attenuated (amplitude modulated) signal is detected by the photodetector 320 at the 625 Hz carrier frequency for each emitter. Because only a single photodetector is used, the photodetector 320 receives all the emitter signals to form a composite time division signal. In the present embodiment, a photodetector is provided which is a sandwich-type photodetector with a first layer which is transparent to infrared wavelengths but detects red wavelengths and a second layer which detects infrared wavelengths. One suitable photodetector is a K1713-05 photodiode made by Hamamatsu Corp. This photodetector provides for detection by the infrared layer of a relatively large spectrum of infrared wavelengths, as well as detection of a large spectrum of wavelengths in the red range by the layer which detects red wavelengths, with a single photodetector. Alternatively, multiple photodetectors could be utilized for the wavelengths in the system.

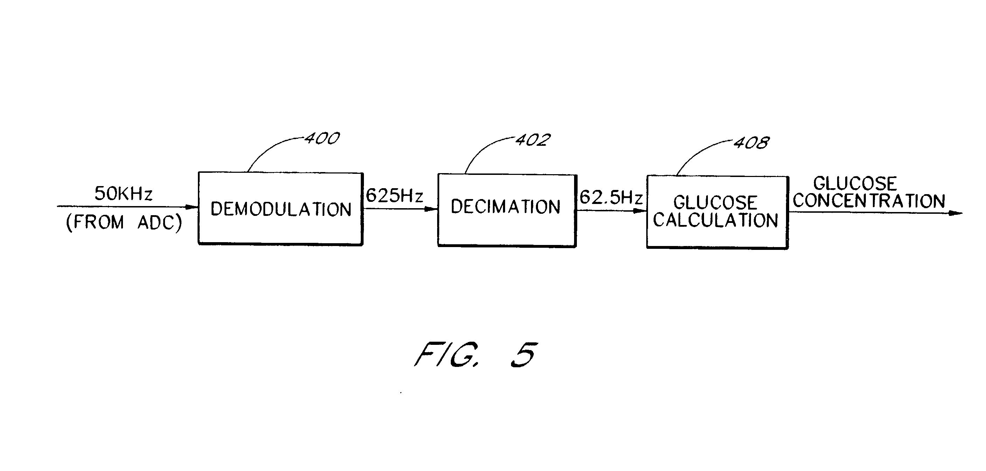

The composite time division signal is provided to the front end analog signal conditioning circuitry 330. Additional detail regarding the front end analog signal conditioning circuitry 330 and the analog to digital converter circuit 332 is illustrated in Figure 3. As depicted in Figure 3, the front end circuity 300 has a preamplifier 342, a high pass filter 344, an amplifier 346, a programmable gain amplifier 348, and a low pass filter 350. The preamplifier 342 is a transimpedance amplifier that converts the composite current signal from the photodetector 320 to a corresponding voltage signal, and amplifies the signal. In the present embodiment, the preamplifier has a predetermined gain to boost the signal amplitude for ease of processing. In the present embodiment, the source voltages for the preamplifier 342 are -15 VDC and +15 VDC. As will be understood, the attenuated signal contains a component representing ambient light as well as the component representing the fight at each wavelength transmitted by each emitter 301 - 305 as the case may be in time. If there is light in the vicinity of the sensor 300 other than from the emitters 301- 305, this ambient light is detected by the photodetector 320. Accordingly, the gain of the preamplifier is selected in order to prevent the ambient light in the signal from saturating the preamplifier under normal and reasonable operating conditions.

The output of the preamplifier 342 couples as an input to the high pass filter 344. The output of the preamplifier also provides a first input 347 to the analog to digital conversion circuit 332. In the present embodiment, the high pass filter is a single-pole filter with a corner frequency of about 1/2 - 1 Hz. However, the corner frequency is readily raised to about 90 Hz in one embodiment. As will be understood, the 625 Hz carrier frequency of the emitter signals is well above a 90 Hz corner frequency. The high-pass filter 344 has an output coupled as an input to an amplifier 346. In the present embodiment, the amplifier 346 comprises a unity gain transimpedance amplifier. However, the gain of the amplifier 346 is adjustable by the variation of a single resistor. The gain of the amplifier 346 would be increased if the gain of the preamplifier 342 is decreased to compensate for the effects of ambient light.

The output of the amplifier 346 provides an input to a programmable gain amplifier 348. The programmable gain amplifier 348 also accepts a programming input from the digital signal processing system 334 on a gain control signal line 343. The gain of the programmable gain amplifier 348 is digitally programmable. The gain is adjusted dynamically at initialization or sensor placement for changes in the medium under test from patient to patient. For example, the signal from different fingers differs somewhat. Therefore, a dynamically adjustable amplifier is provided by the programmable gain amplifier 348 in order to obtain a signal suitable for processing.

The output of the programmable gain amplifier 348 couples as an input to a low-pass filter 350. Advantageously, the low pass filter 350 is a single-pole filter with a corner frequency of approximately 10 Khz in the present embodiment. This low pass filter provides anti-aliasing in the present embodiment.

The output of the low-pass filter 350 provides a second input 352 to the analog-to-digital conversion circuit 332. Figure 3 also depicts additional details of the analog-to-digital conversion circuit. In the present embodiment, the analog-to-digital conversion circuit 332 comprises a first analog-to-digital converter 354 and a second analog-to-digital converter 356. Advantageously, the first analog-to-digital converter 354 accepts signals from the first input 347 to the analog-to-digital conversion circuit 332, and the second analog to digital converter 356 accepts signals on the second input 352 to the analog-to-digital conversion circuitry 332.

In one advantageous embodiment, the first analog-to-digital converter 354 is a diagnostic analog-to-digital converter. The diagnostic task (performed by the digital signal processing system) is to read the output of the detector as amplified by the preamplifier 342 in order to determine if the signal is saturating the input to the high-pass filter 344. In the present embodiment, if the input to the high pass filter 344 becomes saturated, the front end analog signal conditioning circuits 330 provides a '0' output. Alternatively, the first analog-to-digital converter 354 remains unused.

The second analog-to-digital converter 352 accepts the conditioned composite analog signal from the front end signal conditioning circuitry 330 and converts the signal to digital form. In the present embodiment, the second analog to digital converter 356 comprises a single-channel, delta-sigma converter. This converter is advantageous in that it is low cost, and exhibits low noise characteristics. In addition, by using a single-channel converter, there is no need to tune two or more channels to each other. The delta-sigma converter is also advantageous in that it exhibits noise shaping, for improved noise control. An exemplary analog to digital converter is an Analog Devices AD1877JR. In the present embodiment, the second analog to digital converter 356 samples the signal at a 50 Khz sample rate. The output of the second analog to digital converter 356 provides data samples at 50 Khz to the digital signal processing system 334 (Figure 2).

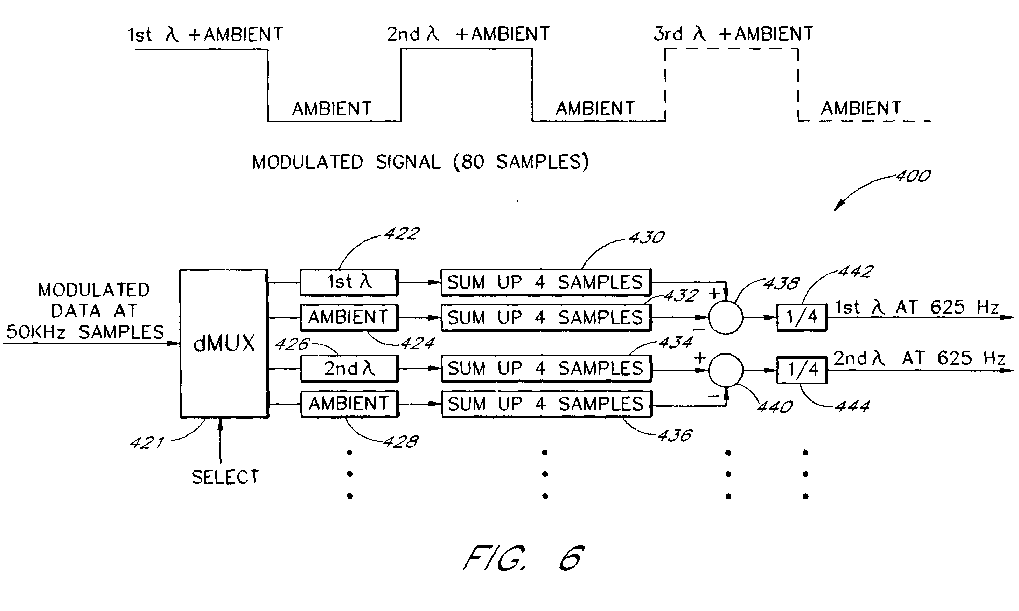

The digital signal processing system 334 is illustrated in additional detail in Figure 4. In the present embodiment, the digital signal processing system comprises a microcontroller 360, a digital signal processor 362, a program memory 364, a sample buffer 366, a data memory 368, a read only memory 370 and communication registers 372. In the present embodiment, the digital signal processor 362 is an Analog Devices AD 21020. In the present embodiment, the microcontroller 360 comprises a Motorola 68HC05, with built in program memory. In the present embodiment, the sample buffer 366 is a buffer which accepts the 50 Khz sample data from the analog to digital conversion circuit 332 for storage in the data memory 368. In the present embodiment, the data memory 368 comprises 32 KWords (words being 40 bits in the present embodiment) of dynamic random access memory.

The microcontroller 360 is connected to the DSP 362 via a conventional JTAG Tap line. The microcontroller 360 transmits the boot loader for the DSP 362 to the program memory 364 via the Tap line, and then allows the DSP 362 to boot from the program memory 364. The boot loader in program memory 364 then causes the transfer of the operating instructions for the DSP 362 from the read only memory 370 to the program memory 364. Advantageously, the program memory 364 is a very high speed memory for the DSP 362.

The microcontroller 360 provides the emitter current control and gain control signals via the communications register 372.

Figures 5-8 depict functional block diagrams of the operations of the glucose monitoring system 299 carried out by the digital signal processing system 334. The signal processing functions described below are carried out by the DSP 362 in the present embodiment with the microcontroller 360 providing system management. In the present embodiment, the operation is software/firmware controlled. Figure 5 depicts a generalized functional block diagram for the operations performed on the 50 Khz sample data entering the digital signal processing system 334. As illustrated in Figure 5, a demodulation, as represented in a demodulation module 400, is first performed. Decimation, as represented in a decimation module 402 is then performed on the resulting data. Then, the glucose concentration is determined, as represented in a Glucose Calculation module 408.

In general, the demodulation operation separates each emitter signal from the composite signal and removes the 625 Hz carrier frequency, leaving raw data points. The raw data points are provided at 625 Hz intervals to the decimation operation which reduces the samples by an order of 10 to samples at 62.5 Hz. The decimation operation also provides some filtering on the samples. The resulting data is subjected to normalization (which essentially generates a normalized AC/DC signal) and then glucose concentration is determined in the Glucose Calculation module 408.

Figure 6 illustrates the operation of the demodulation module 400. The modulated signal format is depicted in Figure 6. The pulses for the first three wavelengths of one full 625 Hz cycle of the composite signal is depicted in Figure 6 with the first tenth cycle being the active first emitter light plus ambient light signal, the second tenth cycle being an ambient light signal, the third tenth cycle being the active second emitter light plus ambient light signal, and the fourth tenth cycle being an ambient light signal, and so forth for each emitter. The sampling frequency is selected at 50 Khz so that the single full cycle at 625 Hz described above comprises 80 samples of data, eight samples relating to the first emitter wavelength plus ambient light, eight samples relating to ambient light, eight samples relating to the second emitter wavelength plus ambient light, eight more samples related to ambient light and so forth until there are eight samples of each emitter wavelength followed by eight samples of ambient light.

Because the signal processing system 334 controls the activation of the light emitters 301-305, the entire system is synchronous. The data is synchronously divided (and thereby demodulated) into the eight-sample packets, with a time division demultiplexing operation as represented in a demultiplexing module 421. One eight-sample packet 422 represents the first emitter wavelength plus ambient light signal; a second eight-sample packet 424 represents an ambient light signal; a third eight-sample packet 426 represents the attenuated second emitter wavelength light plus ambient light signal; and a fourth eight-sample packet 428 represents the ambient light signal. Again, this continues until there is a eight-sample packet for each emitter active period with an accompanying eight-sample packet for the corresponding ambient light period. A select signal synchronously controls the demultiplexing operation so as to divide the time-division multiplexed composite signal at the input of the demultiplexer 421 into its representative subparts or packets.

A sum of the four last samples from each packet is then calculated, as represented in the summing operations 430, 432, 434, 436 of Figure 6. It should be noted that similar operations are performed on the remaining wavelengths. In other words, at the output of the demodulation operation, five channels are provided in the present embodiment. However, only two channels for two wavelengths are depicted in Figure 6 for simplicity in illustration. The last four samples are used from each packet because a low pass filter in the analog to digital converter 356 of the present embodiment has a settling time. Thus, collecting the last four samples from each eight-sample packet allows the previous signal to clear. The summing operations 430, 432, 434, 436 provide integration which enhances noise immunity. The sum of the respective ambient light samples is then subtracted from the sum of the emitter samples, as represented in the subtraction modules 438, 440. The subtraction operation provides some attenuation of the ambient light signal present in the data. In the present embodiment, it has been found that approximately 20 dB attenuation of the ambient light is provided by the operations of the subtraction modules 438, 440. The resultant emitter wavelength sum values are divided by four, as represented in the divide by four modules 442, 444. Each resultant value provides one sample each of the emitter wavelength signals at 625 Hz.

It should be understood that the 625 Hz carrier frequency has been removed by the demodulation operation 400. The 625 Hz sample data at the output of the demodulation operation 400 is sample data without the carrier frequency. In order to satisfy Nyquist sampling requirements, less than 10 Hz is needed (with an active pulse of about 4 Hz in the present embodiment). Accordingly, the 625 Hz resolution is reduced to 62.5 Hz in the decimation operation.

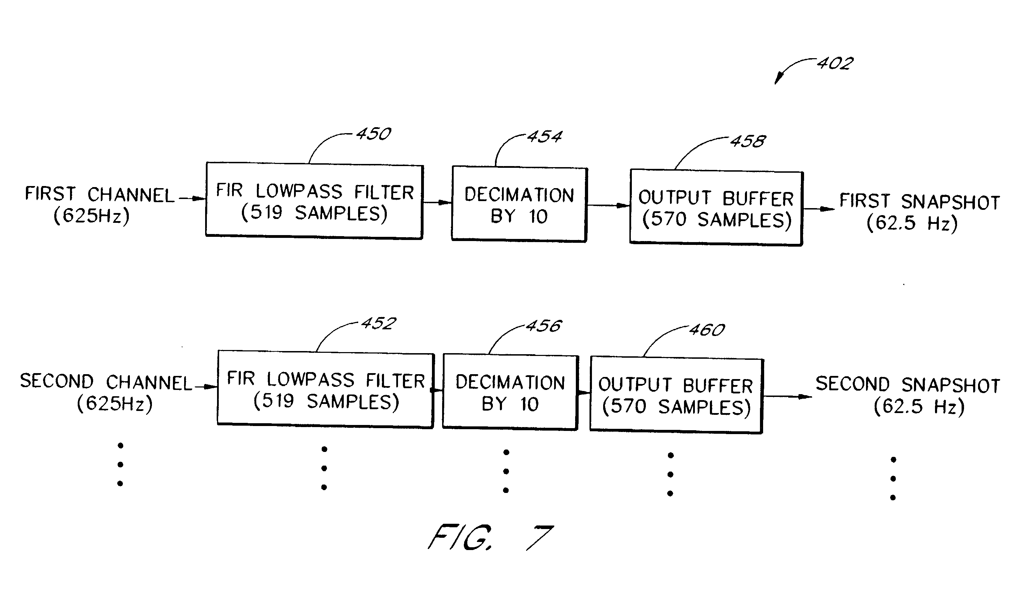

Figure 7 illustrates the operations of the decimation module 402 for the first two wavelengths. The same operations are also performed on the other wavelength data. Each emitter's sample data is provided at 625 Hz to respective buffer/filters 450, 452. In the present embodiment, the buffer/filters are 519 samples deep. Advantageously, the buffer/filters 450, 452 function as continuous first-in, first-out buffers. The 519 samples are subjected to low-pass filtering. Preferably, the low-pass filtering has a cutoff frequency of approximately 7.5 Hz with attenuation of approximately -110 dB. The buffer/filters 450, 452 form a Finite Impulse Response (FIR) filter with coefficients for 519 taps. In order to reduce the sample frequency by ten, the low-pass filter calculation is performed every ten samples, as represented in respective wavelength decimation by 10 modules 454, 456. In other words, with the transfer of each new ten samples into the buffer/filters 450, 452, a new low pass filter calculation is performed by multiplying the impulse response (coefficients) by the 519 filter taps. Each filter calculation provides one output sample for each respective emitter wavelength output buffer 458, 460. In the present embodiment, the output buffers 458, 460 are also continuous FIFO buffers that hold 570 samples of data. The 570 samples provide respective samples or packets (also denoted "snapshot" herein) of samples. As depicted in Figure 5, the output buffers provide sample data for Glucose Calculation Module 408 for two wavelengths.

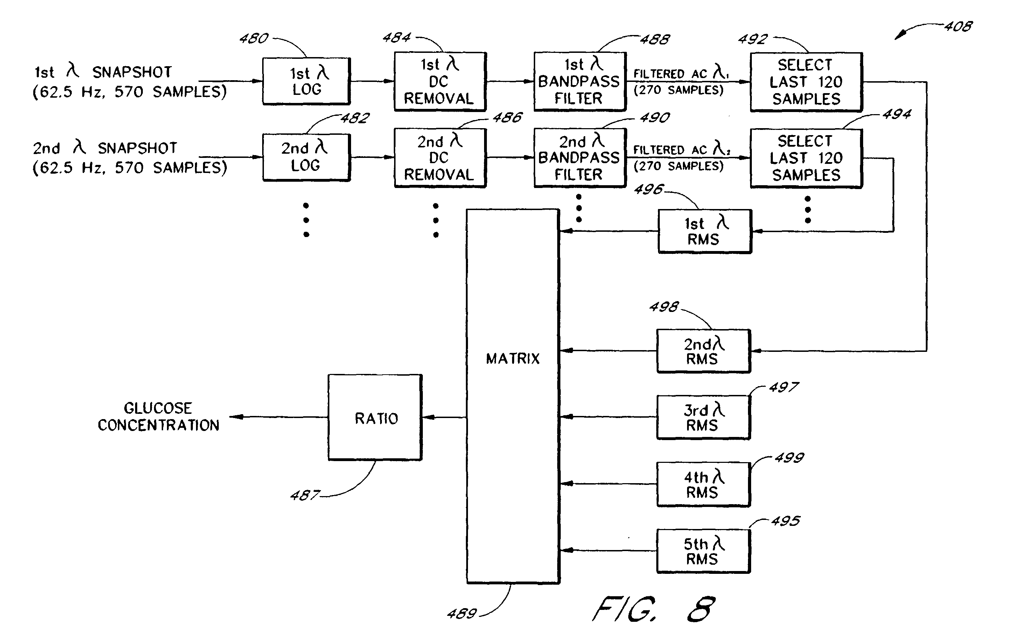

Figure B illustrates additional functional operation details of the Glucose Calculation module 408. As represented in Figure 8, the Glucose Calculation operation accepts packets of samples for each wavelength (e.g., 570 samples at 62.5 Hz in the present embodiment) representing the attenuated wavelength signals, with the carrier frequency removed. The respective packets for each wavelength signal are normalized with a log function, as represented in the log modules 480, 482. Again, at this point, only two channels are illustrated in Figure 8. However, in the present embodiment, five channels are provided, one for each wavelength. The normalization effectively creates an AC/DC normalized signal, this normalization is followed by removal of the DC portion of the signals, as represented in the DC Removal modules 484, 486. In the present embodiment, the DC removal involves ascertaining the DC value of the first one of the samples (or the mean of the first several or the mean of an entire snapshot) from each of the respective wavelength snapshots, and removing this DC value from all samples in the respective packets.

Once the DC signal is removed, the signals are subjected to bandpass filtering, as represented in Bandpass Filter modules 488, 490. In the present embodiment, with 570 samples in each packet, the bandpass filters are configured with 301 taps to provide a FIR filter with a linear phase response and little or no distortion. In the present embodiment, the bandpass filter has a narrow passband from 3.7 - 4.3 Hz. This provides a narrow passband which eliminates most noise and leaves the portion of the signal due to the active pulse. The 301 taps slide over the 570 samples in order to obtain 270 filtered samples representing the filtered signal of the first emitter wavelength and 270 filtered samples representing the filtered signal of the second emitter wavelength, continuing for each emitter wavelength. In an ideal case, the bandpass filters 488, 490 assist in removing the DC in the signal However, the DC removal operation 484, 486 also assists in DC removal in the present embodiment.

After filtering, the last 120 samples from each packet (of now 270 samples in the present embodiment) are selected for further processing as represented in Select Last 120 Samples modules 492, 494. The last 120 samples are selected in order to provide settling time for the system.

The RMS for the samples is then determined for each of the 120-sample packets (for each wavelength). The process to obtain the overall RMS values is represented in the RMS modules 495 - 499.

The resultant RMS values for each wavelength provide normalized intensity values for forming equations according to Beer-Lambert's law. In other words, for Beer-Lambert equation

$$I = I_o e^{-(pl \cdot c_1 \cdot \varepsilon_1 + pl \cdot c_2 \cdot \varepsilon_2 + etc.)} \tag{3}$$

then taking the log of operations 480 - 482:

$$1n(I) = 1n(I_o) - (pl \cdot c_i \cdot \varepsilon_1 + pl \cdot c_2 \cdot \varepsilon_1 + etc.) \tag{4}$$

Then performing DC removal though the DC removal operations 484, 486 and Band pass filter operations 488, 490, the normalized equation becomes:

$$I_{norm\lambda}=-pl \cdot c_1 \cdot \varepsilon_1 + pl \cdot c_2 \cdot \varepsilon_2 + etc. \tag{5}$$

The RMS values (blocks 495 - 499) for each wavelength provide $I_{norm\lambda}$ for the left side of Equation (7). The extinction coefficients are known for the selected wavelengths.

As will be understood, each equation has a plurality of unknowns. Specifically, each equation will have an unknown term which is the product of concentration and pathlength for each of the constituents of concern (hemoglobin, oxyhemoglobin, glucose and water in the present embodiment). Once a normalized Beer-Lambert equation is formed for each wavelength RMS value (the RMS value representing the normalized intensity for that wavelength), a matrix is formed as follows:

$$I_{norm\lambda_1}=-(\varepsilon_{1\lambda_1}c_1+\varepsilon_{2\lambda_1}c_2+\varepsilon_{3\lambda_1}c_3+\varepsilon_{4\lambda_1}c_4+\varepsilon_{5\lambda_1}c_5)\,pl \tag{6}$$

$$I_{norm\lambda_2}=-(\varepsilon_{1\lambda_2}c_1+\varepsilon_{2\lambda_2}c_2+\varepsilon_{3\lambda_2}c_3+\varepsilon_{4\lambda_2}c_4+\varepsilon_{5\lambda_2}c_5)\,pl \tag{7}$$

$$I_{norm\lambda_3}=-(\varepsilon_{1\lambda_3}c_1+\varepsilon_{2\lambda_3}c_2+\varepsilon_{3\lambda_3}c_3+\varepsilon_{4\lambda_3}c_4)+\varepsilon_{5\lambda_3}c_5)pl \tag{8}$$

$$I_{norm\lambda_4}=-(\varepsilon_{1\lambda_4}c_1+\varepsilon_{2\lambda_4}c_2+\varepsilon_{3\lambda_4}c_3+\varepsilon_{4\lambda_4}c_4\varepsilon_{5\lambda_4}c_5)pl \tag{9}$$

$$I_{norm\lambda_5}=-(\varepsilon_{1\lambda_5}c_1+\varepsilon_{2\lambda_5}c_2+\varepsilon_{3\lambda_5}c_3+\varepsilon_{4\lambda_5}c_4\varepsilon_{5\lambda_5}c_5)pl \tag{10}$$

where

$C_1$ = concentration of water
$C_2$ = concentration of hemoglobin
$C_3$ = concentration of oxyhemoglobin
$C_4$ = concentration of Glucose
$C_5$ = concentration of Glucose

and

$\varepsilon_{1\lambda n}$ = extinction coefficient for water at $\lambda n$
$\varepsilon_{2\lambda n}$ = extinction coefficient for hemoglobin at $\lambda n$
$\varepsilon_{3\lambda n}$ = extinction coefficient for oxyhemoglobin at $\lambda n$
$\varepsilon_{4\lambda n}$ = extinction coefficient for Glucose at $\lambda n$
$\varepsilon_{5\lambda n}$ = extinction coefficient for Glucose at $\lambda n$

The equations are solved using conventional matrix algebra in order to solve for the product of concentration times pathlength for each constituent, as represented in the Matrix block 489.

In order to remove the path length term, in the present embodiment where glucose is desired, a ratio is performed of the product of pathlength times concentration for glucose to the product of pathlength times the concentration of water as represented in a ratio block 487. Since the pathlength is substantially the same for each wavelength due to normalization (i.e., taking AC/DC) and due to minimal perturbation (e.g., 10%), the pathlength terms cancel, and the ratio indicates the concentration of glucose to water (preferably, this is scaled to mg/dL). The glucose concentration is provided to the display 336.

It should be noted that it may also be possible to create an empirical table by way of experiment which correlates ratios of one or more of the concentration times path length terms to blood glucose concentration.

Even with the emitter driver circuit of Figure 2A discussed above, infrared LEDs with the longer wavelengths are also inherently unstable with respect to their power transmission. Accordingly, in one advantageous embodiment, the instabilities for the source LEDs can be corrected to accommodate for the instabilities depicted in Figure 2C. As illustrated in Figure 2C, two curves are depicted representing transmitted power over time. A first curve labelled AA represents power transmission from LEDs having wavelengths of 660 nm and 905 nm. As illustrated, these emitters have relatively stable power transmission over time.

A second curve labelled BB represents power transmission from an emitter with a wavelength of approximately 1330 nm. As illustrated, typical emitters of this wavelength have unstable power transmission over time.

Accordingly, in one embodiment, the emitters in the 1300 nm range are selected as with an integrated photodetector. An appropriate laser diode is an SCW-1300-CD made by Laser Diode, Inc. An appropriate LED is an Apitaxx ETX1300T. With such an emitter, a configuration as depicted in Figure 2B can be used, whereby the internal photodiode in the emitter is also sampled to detect the initial intensity $I_0$ times a constant ($\alpha$). In general, the signal detected after transmission through the finger is divided by the $\alpha I_o$ signal. In this manner, the instability can be normalized because the instability present in the attenuated signal due to instability in the emitter will also be present in the measured $\alpha I_o$ signal.

Figure 2B depicts such an embodiment illustrating only one emitter 301 (of the emitters 301-305). However, all or several of the emitters 301-305 could be emitters having an internal photodiode. As depicted in Figure 28, the emitter 301 has an internal photodiode 301a and its LED 301b. As depicted in Figure 2B, light emitted from the LED 301b in the emitter 301 is detected by a photodiode 301a. The signal from the photodiode 301a is provided to front end analog signal conditioning circuitry 330A. The analog signal conditioning circuitry 330A is similar to the analog signal conditioning circuitry 330. However, because the photodiode 301a detects a much stronger intensity compared to the detector 320 (due to attenuation by tissue), different amplification may be required.

After analog signal conditioning in the front end analog signal conditioning circuitry 330A, the signal from the photodiode 301a is converted to digital form with an analog to digital conversion circuit 332a. Again, it should be understood that the analog to digital conversion circuit 332a can be the same configuration as the analog to digital conversion circuit 332. However, because the signal from the photodiode 301 a and the detector 320 appear at the same time, two channels are required.

The attenuated light signal through the finger is detected with the detector 320 and passed through front end analog signal conditioning circuit 330 and is converted to digital form in analog to digital conversion circuit 332, as described in further detail below. The signal representing the intensity of the light transmitted through the finger 310 is divided as represented by the division block 333 by the signal which represents the intensity of light from the LED 301b detected by the photodiode 301a.

In this manner, the variations or instability in the initial intensity $I_0$ cancel through the division leaving a corrected intensity which is divided by the constant $\alpha$. When the log is performed as discussed below, and bandpass filtering is performed, the constant $\alpha$ term is removed leaving a clean signal.

Mathematically, this can be understood by representing the attenuated signal under Beer-Lambert's Law and the signal from the photodiode 301 a as $\alpha I_o$ as discussed above:

Thus, the signal emerging from the analog to digital conversion circuit 332 is as follows:

$$I = I_o e^{\Sigma(-\varepsilon \cdot c \cdot pl)}$$

Dividing Equation 3 by $\alpha I_o$ and simplifying provides the signal after the division operation 333:

$$= \frac{e^{\Sigma-(\varepsilon \cdot c \cdot pl)}}{\alpha}$$

Thus providing a normalized intensity signal for the input to the digital signal processing circuit 334.

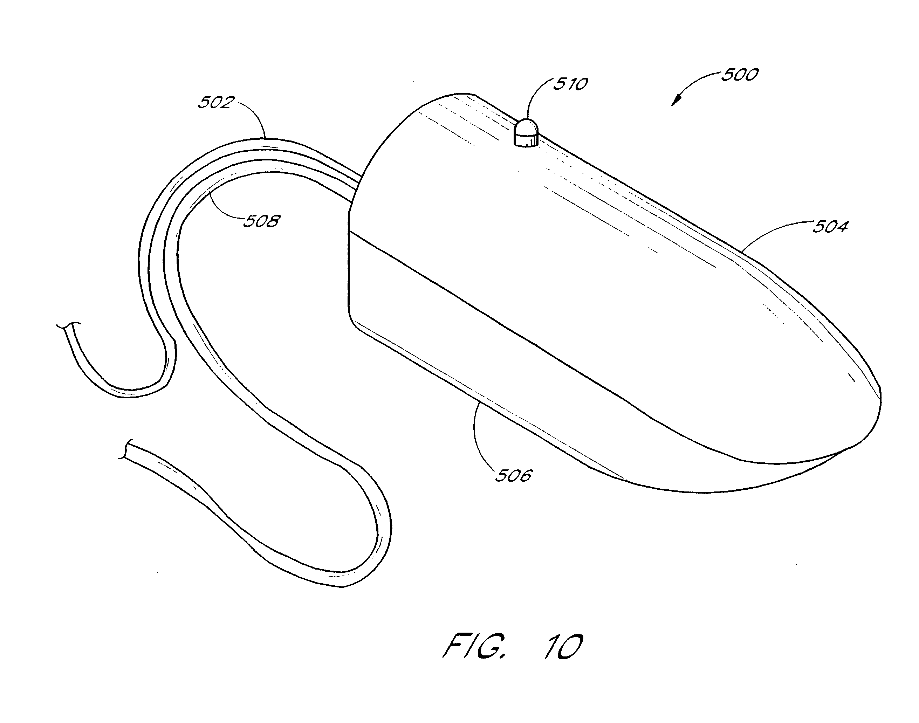

Figure 10 depicts a perspective view of one alternative embodiment of an inflatable bladder sensor 500 which can be used to induce an active pulse in accordance with the teachings of the present invention. This inflatable bladder sensor 500 is for a bed-side blood glucose monitor. The inflatable bladder sensor 500 has electrical connections 502 for coupling the device to the blood glucose system 299. Typically, the electrical connection 502 carries sufficient conductors to power the emitters 301 - 305 and to receive a detector signal from the detector 320.

The inflatable bladder sensor 500 has a curved upper surface 504 and vertical sides 506. The inflatable bladder sensor 500 also has an fluid pressure supply tube 508. In one advantageous embodiment, the supply tube cycles air into and out of an inflatable bladder within the inflatable bladder sensor 500. The fluid supply tube 508 couples to the bedside glucose monitoring system which is equipped with a cycling pump to induce pressure and remove pressure from the supply tube 508. In one embodiment, a pressure relief valve 510 is located on the upper surface 504 to allow release of pressure in the inflatable bladder.

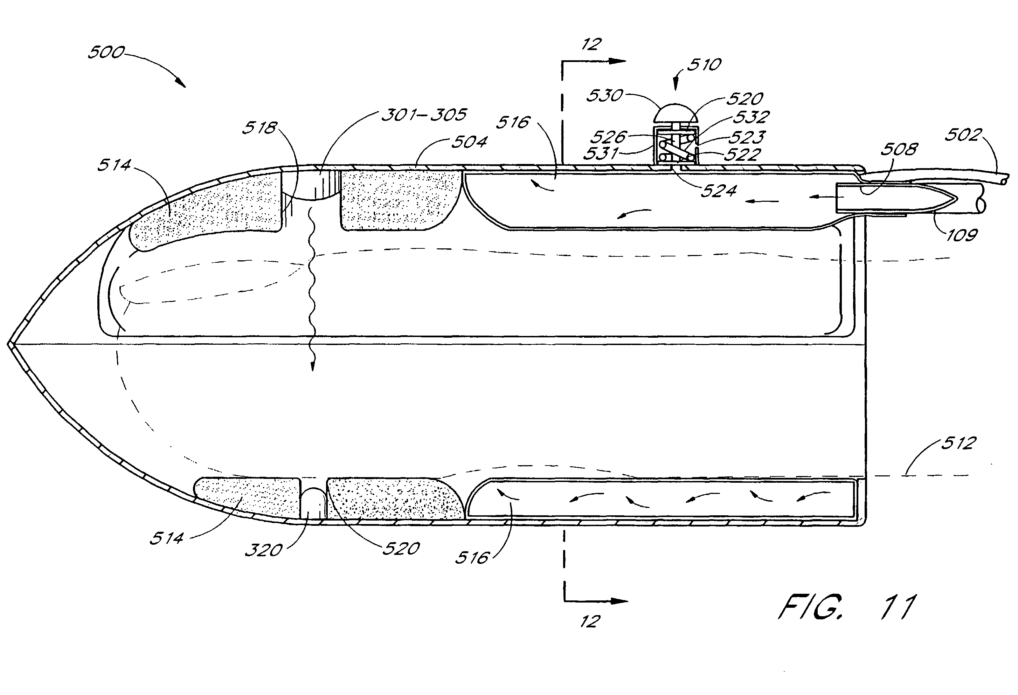

Figure 11 depicts a cross-sectional view along the inflatable bladder sensor 500 of Figure 10. As depicted in Figure 11, a human digit or finger 512 is positioned inside the sensor 500. The finger 512 is supported by a pad 514 in the area of light transmission. A flexible inflatable bladder 516 surrounds the finger proximally from the area of light transmission. The pad has an aperture 518 to enable emitters 301 - 305 to provide unobstructed optical transmission to the surface of finger 512.

Surrounded by the padding 514 and opposite the emitters 301-305 is the detector 320. The detector 320 is positioned within an aperture 520 in the pad 514 to ensure that photodetector is separated from the finger 512. A serpentine arrow is shown extending from the light emitters 301 - 305 to the detector 320 to illustrate the direction of propagation of light energy through the finger 512.

Relief valve 510 enables manual and automatic release of pressure in the inflatable bladder 516. Relief valve 510 has a valve plate 522 which is spring biased to seal an aperture 524. The valve plate is connected to relief valve shaft 526. A valve button 530 is coupled to the valve shaft. The valve shaft extends through a valve housing 530 which forms a cylindrical sleeve shape. The valve housing is coupled to the upper surface 504 of sensor 500. The valve housing has an aperture 523 which allows air to readily escape from the relief valve. Preferably, the relief valve is designed to ensure that the pressure is not high enough to cause damage to nerves. Accordingly, if the pressure increases beyond a certain point, the relief valve allows the excess fluid to escape, thereby reducing the pressure to the maximum allowable limit. Such pressure relief valves are well understood in the art. Relief valve 510 could also be a spring-loaded needle-type valve.

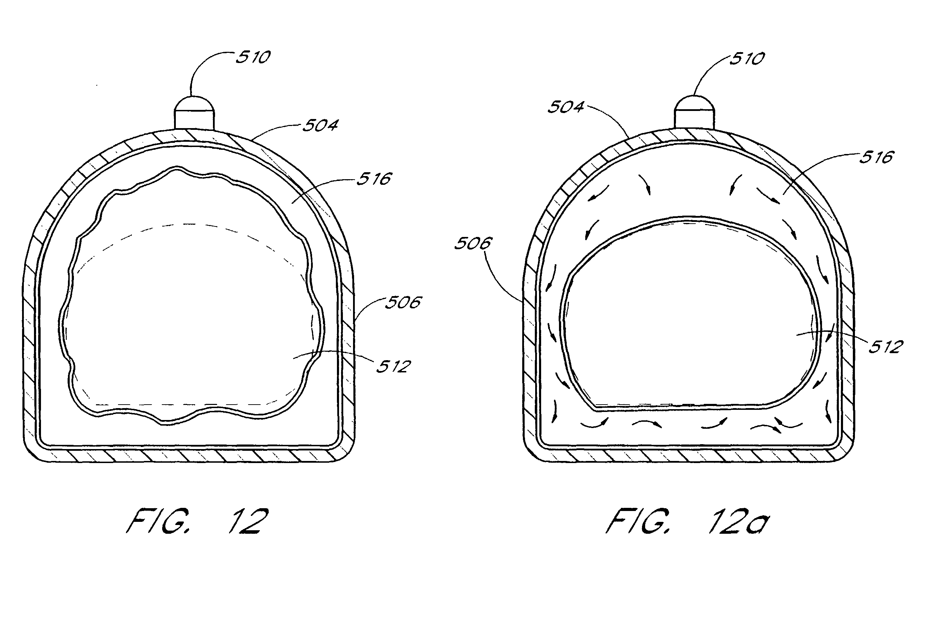

Figure 12 depicts a sectional view along line 12-12 of Figure 11 to illustrate the state of the sensor 500 when the inflatable bladder 516 is deflated. Figure 12a depicts the same sectional view as Figure 12 with the bladder 516 inflated.

With this configuration, the blood glucose system can cycle fluid into and out of the inflatable bladder 516 at the selected rate to actively induce a pulse of sufficient magnitude as discussed above.

Additional Application of Active Pulse

As discussed in the WO 96/12435 a saturation transform may be applied to each 120 sample packet. It has been found that a second maximum representing venous oxygen saturation exists in the Master Power Curve during motion of the patient. In view of this, it is possible to utilize the inducement of a pulse disclosed herein through physically perturbing the medium under test in order to obtain the second maxima in the Master Power Curve, and thereby obtain the venous oxygen saturation if desired. The modulation may be lower than 10% because hemoglobin and oxyhemoglobin concentrations are higher than glucose and absorption at 660 nm and 905 nm are relatively strong. Thus, modulation from 1-5% may provide adequate results.

Filter Wheel

As mentioned above, a filter wheel 110A can be a conventional dichroic filter or a filter made in accordance with the following methods.

Figure 14 shows an exemplary dichroic filter fabricated according to conventional methods. Previous methods employed to fabricate such optical filters typically involved laying out a circular substrate and then selectively increasing the coating thicknesses on the surface of the circular substrate.

Such a filter 1150 is depicted in Figure 14 as having coating layers 1152, 1154, 1156, etc., of increasing thicknesses to form a spiral configuration as the filter 1150 is rotated. It should be understood that the coating thicknesses depicted in Figure 14 are exaggerated for ease of illustration. This method of optical coating is carried around substantially the entire circumference of the circular substrate so that as the coated substrate revolves, the thickness of the optical coating grows throughout the entire revolution and then suddenly drops back from the thickest coating to the thinnest coating at the end of one revolution.

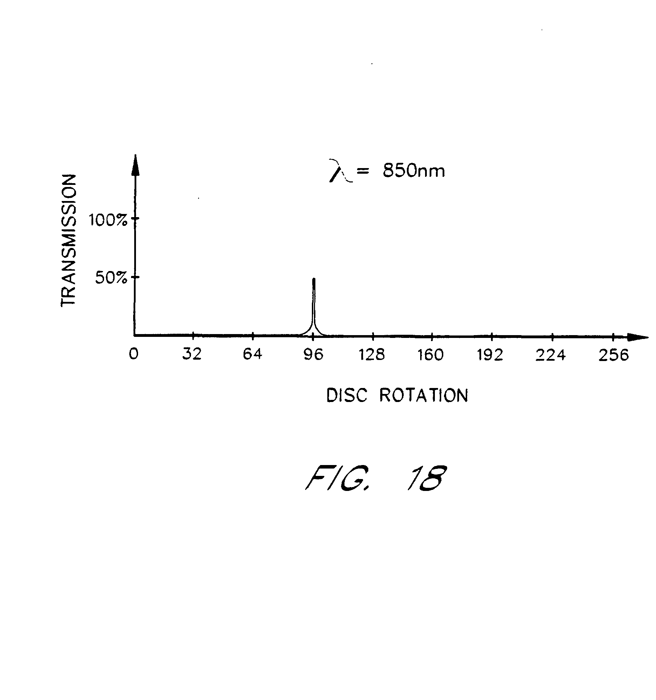

In addition, conventional filters of the type depicted in Figure 14 generally have many layers (e.g., 100 or more layers is common). The number of layers in conventional filters are provided to provide very precise pass bands (for a bandpass filter). Figure 18 depicts an exemplary transmission characteristic for a conventional rotational dichroic filter versus degrees of rotation for a selected wavelength. As illustrated in Figure 18, the pass band of the filter is very precise for the selected wavelength, generally without side-lobes, and also provides essentially zero transmission outside the pass band. A very high number of layers is required to obtain a filter with this near ideal precision. It should be understood, that this very narrow passband is in different rotational positions for different wavelengths. In other words, a conventional dichroic filter can be characterized as a monochrometer which passes a different wavelength at different rotational positions.

Creating each layer is expensive due to the continuous rotational variation from thin to thicker. Thus, when many layers are created (e.g., 100 or more for good precision), such conventional filters are very costly.

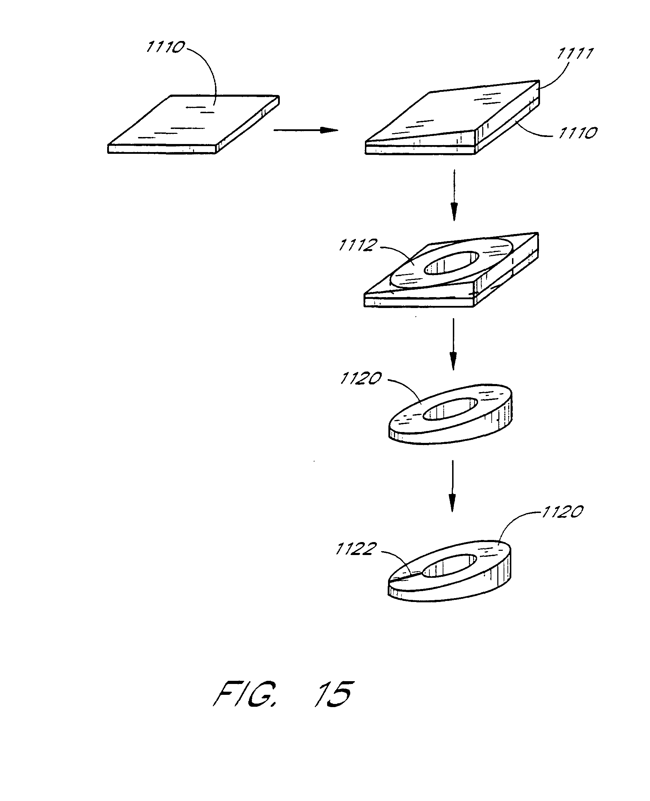

In accordance with the present invention, a dichroic filter is disclosed which differs significantly from conventional dichroic filters. Figure 15 depicts a filter 1120 along with the steps followed in the method of producing a filter.

The dichroic filter is made in a novel manner in which the multiple optical coatings are created on a substrate to form a wedge-like substrate. For a rotational filter, the substrate is then cut to form a rotational disk filter.

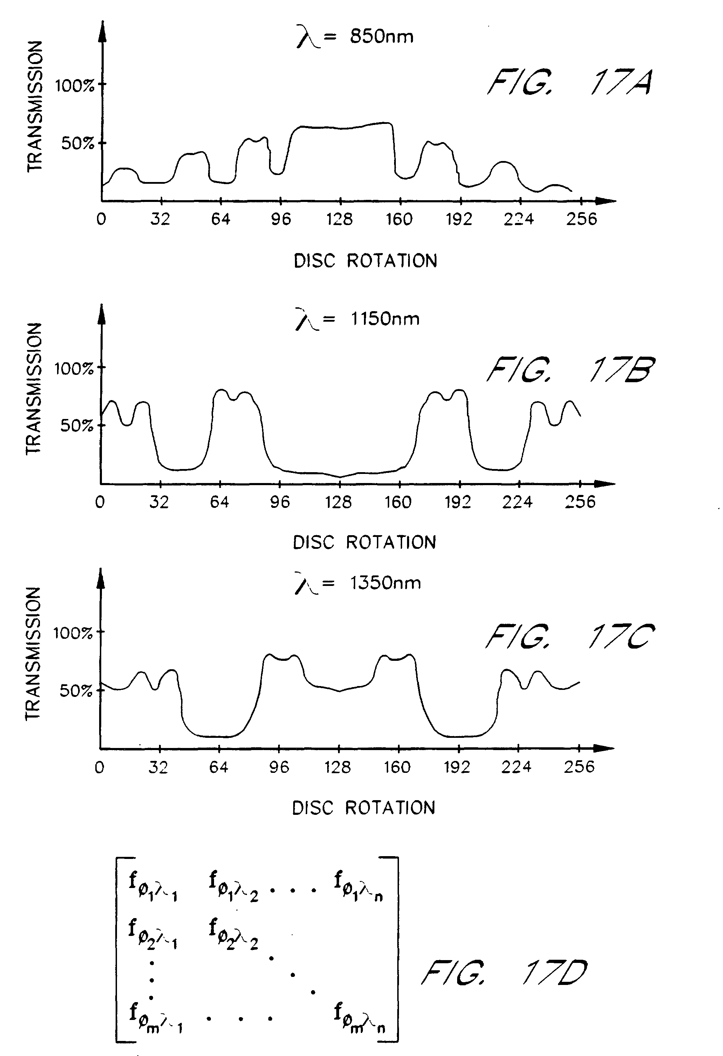

In addition, the dichroic filter has fewer layers than conventional filters. This provides for less precision in the transmission characteristic of the filter. Figure 4A - 4C depict the optical transmission characteristics for selected wavelengths of an exemplary rotational filter having only 17 optical coating layers. As illustrated in Figures 17A - 17C, the transmission characteristic is not as precise as the transmission characteristic of the filter represented in Figure 18. As depicted in Figures 17A-17C, the dichroic filter has several pass-bands for each wavelength depicted. In addition, outside the pass-bands, the transmission does not fall completely to zero, as with the conventional precision filters. The reduced precision in the passbands is due to the reduced number of layers in the filter. It should be understood, that the reduced precision explained above is not limited to rotational dichroic filters, but could also be advantageous with dichroic filters that are vibrated (e.g., through oscillation or the like), and for any other optical filter which conventionally involves high precision in the pass-bands. The decreased precision of the filter of the present invention is accommodated with signal processing as further explained below to obtain the required precision. In this manner, the cost of the filter can be reduced.

When both aspects of the filter in accordance with the present invention are used (layering process and reduced number of layers), the resulting filter is much less expensive to construct than conventional dichroic filters. However, it should be noted that using either aspect of reducing cost is advantageous in itself. For instance, a conventional rotational filter could be fabricated with far fewer layers, but using conventional layering techniques such that the filter increases in thickness through the entire revolution of the filter. Alternatively, the method of fabrication disclosed herein could be used to form a rotational filter with conventional precision (e.g., many layers) at reduced manufacturing costs due to the improved manufacturing method.

In the method which reduces the cost of layering the optical filter, a flat substrate 1110 (Figure 15) is coated with optical coatings of increasing thickness to form a wedge-shaped coated layer 1111. It should be noted that for purposes of clearly illustrating the present invention, the thickness of the optical coating 1111 has been exaggerated, and in practical applications the thickness of the optical layer 1111 varies from roughly 1.66 micrometers to about 3.33 micrometers, with an average thickness of about 2.35 micrometers. It should also be understood that these thicknesses are approximate and may vary depending upon the index of refraction of the layer materials. Therefore, the optical coatings which define the filter are applied across a substrate rather than continually applying coatings circumferentially, thus, significantly reducing the cost of the filter. The filter at this point provides a dichroic filter which could be used in oscillating filter type applications.

For a rotational filter, once the optical layers 1111 have been applied to the substrate 1111, a cylindrical portion 1112 is cut from the wedge-shaped slab formed by the optical layer 1111 together with the substrate 1110. A cylindrical aperture is then formed in the center of the cylindrical portion 1112 to form a mounting hole. In certain applications, it is desirable to form an optically opaque strip such as a brass strip 1122 over a portion of the optical filter disk 1120. The brass strip provides a zero-transmission reference portion of the disc 1120 which may be helpful for noise cancellation in certain signal processing applications.

The above description provides ease of illustration for understanding one aspect of the present invention. However, it should be understood that the method may, in practice, involve first cutting the substrate into a disk. Thereafter, the optical coatings are applied onto the disk as though the disk were still square so that the excess falls onto the platform (not shown) supporting the disk within the vacuum tank. In this manner the wedge is formed on the surface of the disk 1120 as shown in Figure 15.

It will be understood that the disk 1120 does not continually increase in thickness through the entire-circumference of the wheel, but increases in thickness and then decreases in thickness. However, both halves of the circumference can be utilized as further described below.

In addition to the reduced manufacturing cost of the filter described above, a minimal number of optical coating layers are deposited. In one preferred embodiment, only 17 layers are necessary to obtain the desired resolution.

Although reducing the number of layers results in less precise filters, such imperfections can be accommodated in digital signal processing steps. For example, as explained above, conventional dichroic filters typically pass a single frequency band at a time (Figure 18), while the filter of the preferred embodiment may allow for multiple bands to pass, since this is accounted for, and can be compensated through signal processing.

It should be noted here that the resolution typically necessary for applications involving more expensive interferometers or monochrometers is typically not necessary for analyzing liquids. However, additional layers can be added at greater spacing intervals in order to increase resolution of the filter.

## COMPENSATING DIGITAL SIGNAL PROCESSING

As briefly set forth above, the imprecision of a filter made in accordance with the present invention having a minimal number of optical coatings can be accommodated through signal processing.

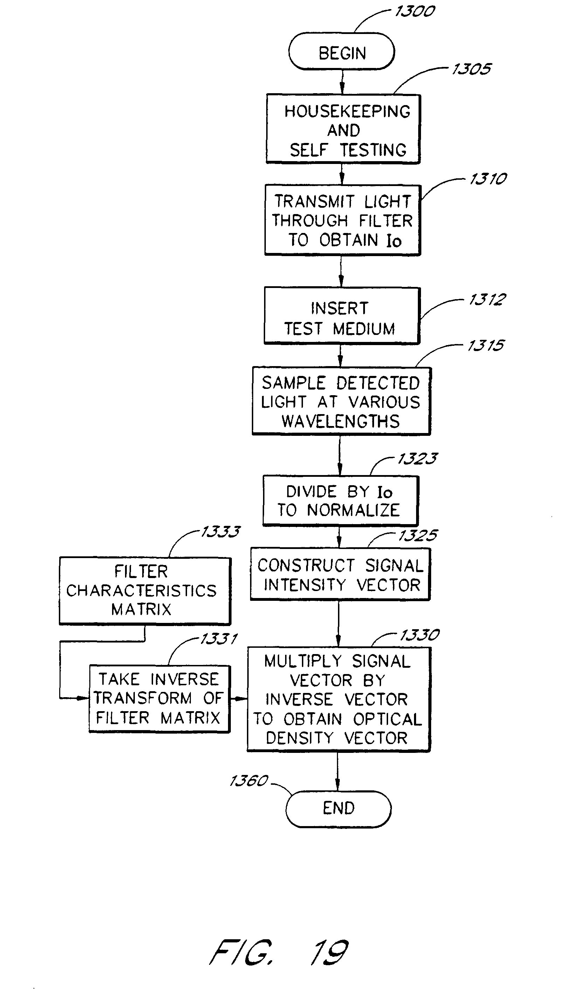

Figure 19 is a data flow diagram which details the method used to compensate for the imprecision of the filter made in accordance with the present invention. It should be understood, however, that prior to run-time, initialization is performed.

PRE-RUN-TIME INITIALIZATION

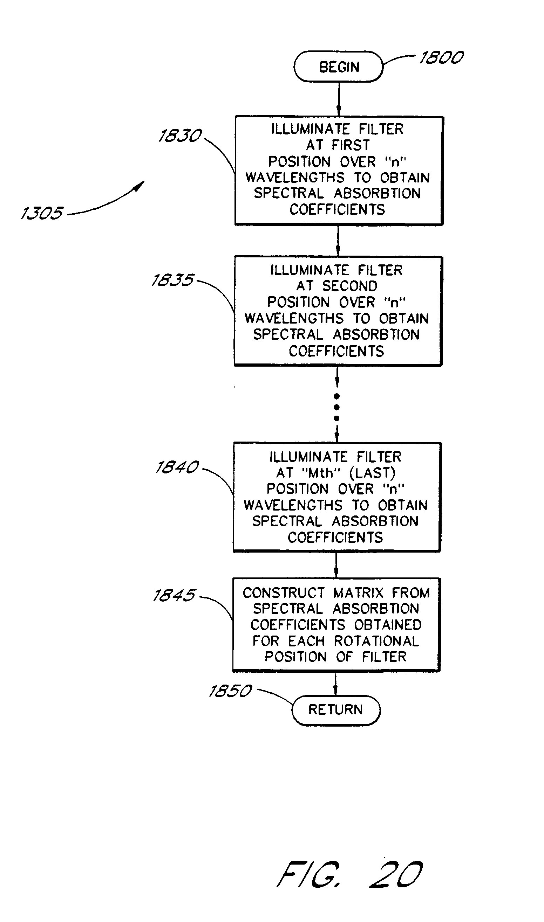

The initialization is performed at the factory or other time prior to use. In general, a filter characteristics matrix is constructed, as described in greater detail below with reference to Figure 20. The filter characteristics matrix represents the transmission characteristics of the dichroic filter 1120 at different portions of the filter 1120 and for various wavelengths of light. The filter characteristics matrix is used in order to extract portions of the electrical signal generated by a detector which are due simply to the optical attenuation caused by the filter 1120. In other words, by knowing the filter characteristics, the imprecision of the filter can be accounted for.

The filter characteristic matrix is a two-dimensional matrix. The filter characteristic matrix includes one column for each wavelength of light which is characterized and one row for each position (rotational in the present invention) of the filter 1120, at which characterization (of the filter characteristic) is performed. Thus, in one embodiment, the filter characteristic matrix includes 16 columns and 256 rows when 16 wavelengths are characterized and 256 positions of the filter 1120 are defined. It should be understood here that it is not necessary that 16 different wavelengths be used: the use of additional wavelengths is particularly advantageous for increasing the signal-to-noise ratio. Since about half of the incident light is transmitted through the filter at each position of the filter, the same wavelength is detected multiple times (although in a unique combination with other wavelengths each time) so that the overall signal intensity is from 10 to 100 times the intensity of any single wavelength and much higher than the noise floor. This is commonly referred to as Felgate's advantage. In this manner the spectral response of the entire filter 1120 over the expected measured wavelengths is completely characterized. The method employed to construct the filter characteristics matrix is described in detail below with reference to Figure 20.

DERIVATION OF THE FILTER CHARACTERISTIC MATRIX

Figures 17A-17D, together with Figure 20, illustrate in greater detail, the method employed to obtain the filter characteristic matrix. The derivation routine is illustrated in Figure 20 and starts with a begin block 1800.

The activity blocks 1830-1845, together with Figures 17A-17D, illustrate the method used in accordance with the present invention to construct the filter characteristics matrix. The filter 1120 reflects and transmits optical radiation in different proportions for different wavelengths at different places on the filter disk 1120. This is clearly illustrated in Figure 17A-17C, wherein Figure 17A represents the optical transmission of light at a wavelength of 850 nanometers plotted versus each of a possible 256 disk rotational positions (for one embodiment). As shown in Figure 17A, when the disk 1120 is in the initial starting position (i.e., $\phi = 0$ where 0 represents the rotational position of the filter 1120), the transmission of light at 850 nanometers is approximately 10% through the filter 1120, while when the disk 1120 is rotated so that $\phi = 32$, the optical transmission of light at 850 nanometers through the filter 120 is approximately 25%. Again, between the disk rotational positions of $\phi = 128$ to $\phi = 160$, the transmission of light at 850 nanometers wavelength through the filter 1120 is approximately 75%. Thus, the optical transmission for $\lambda = 850$ nanometers is entirely characterized over 256 rotational positions of the disk filter 1120, as depicted in Figure 17A.

Figure 17B depicts the optical transmission characteristics of light at 1,150 nanometers over the same 256 rotational positions of the disk 1120. Similarly, Figure 17C depicts a plot of the optical transmission of light at 1,350 nanometers through the disk filter 1120 at each of the 256 rotational positions of the disk 1120. In one actual embodiment of the invention, the optical transmission characteristics of the filter 1120 are described for 256 rotational positions at each of 16 wavelengths between 850 nanometers and 1,400 nanometers.

Thus, from these measurements, a filter characteristic matrix may be constructed, as shown in Figure 170. The filter characteristic matrix designated in Figure 170 as $F(\phi,\lambda)$ includes 256 rows and 16 columns. Each column of the filter characteristic matrix comprises the spectral transmission characteristics of the disk 1120 at each of the 256 rotational positions of the disk 1120 for the selected wavelength for that column.

In order to construct the filter characteristic matrix depicted in Figure 170, the filter 1120 is illuminated at a first rotational position over each of the 16 wavelengths to obtain spectral transmission coefficients for each of the 16 wavelengths, as indicated within an activity block 1830. Once the spectral transmission coefficients have been determined for the first rotational position as indicated within the activity block 1830, the filter is illuminated at a second rotational position (i.e., $\phi = 1$) over the 16 selected wavelengths to obtain spectral transmission coefficients for the second rotational position, as represented in an activity block 1835. This method is carried on for each of the possible rotational positions of the disk 1120 until, as indicated within an activity block 1840, the filter is illuminated at the "mth," or last, rotational position (i.e., position 256) of the disk filter 1120 over the 16 selected wavelengths to obtain the spectral transmission coefficients for the last rotational position. In one preferred embodiment, where a stepper motor is used, the rotational positions will be precise from revolution to revolution of the disk 1120. Of course, a computer disc motor with salient poles and run at a constant speed could be used provided that phase dithers are minimized to less than one part in 256.

Once spectral transmission coefficients have been determined for all 16 wavelengths of all 256 rotational positions of the disk 1120, the filter characteristics matrix is constructed, as indicated within an activity block 1845. The matrix is defined by column and row where columns represent coefficients and row represents the wavelength by putting coefficients. Once the filter characteristics matrix is constructed, the system has the necessary constraints for processing.

It should be understood that derivation of a filter characteristic matrix has been described for purposes of the rotational filter 1120. However, an oscillating filter, or any filter with defined positions on the filter such as Fabry-Perot type filters and even fixed filters such as those used in CCD applications can also be characterized in accordance with the discussion above.

RUN-TIME PROCESSING

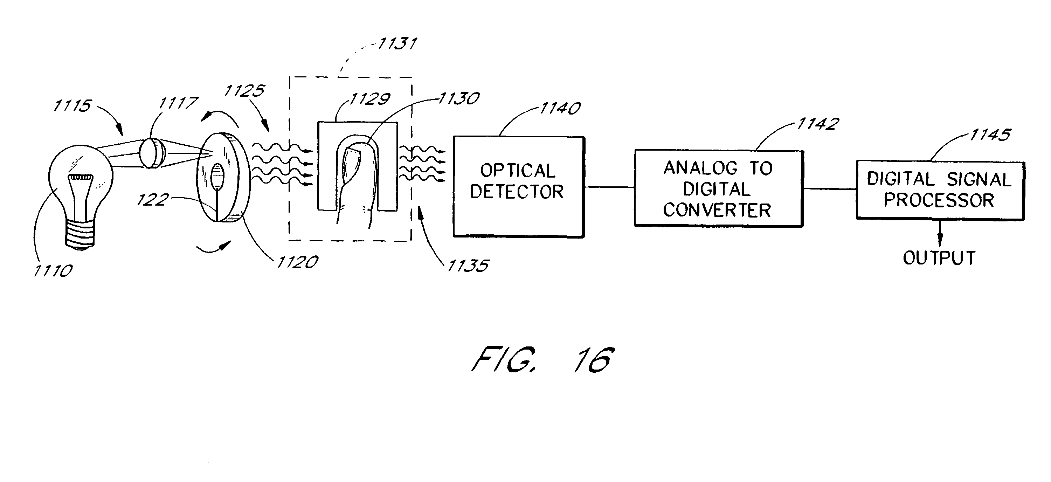

Discussion of the overall processing in order to account for imprecision of the filter through the use of the filter characterization matrix is made with reference to Figures 16, 20 and 21.

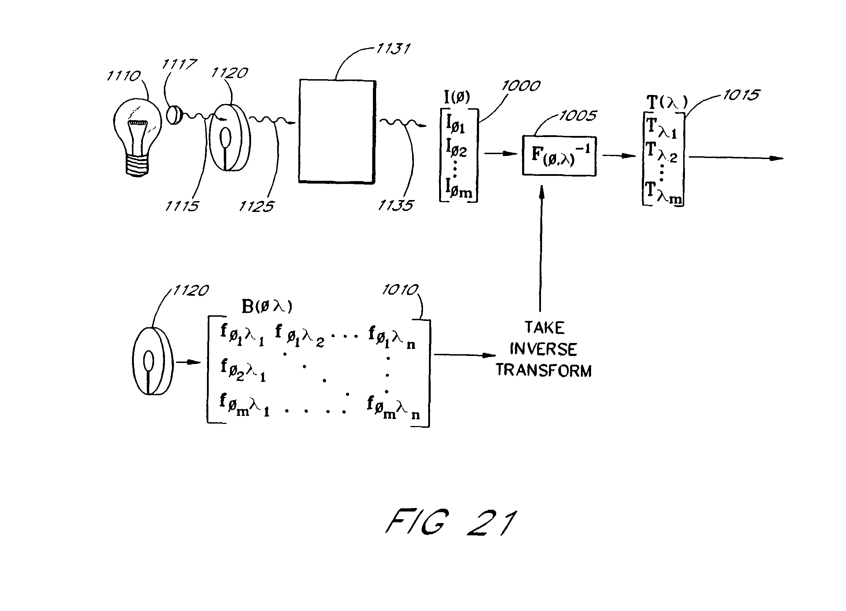

Figure 16 illustrates the use of the filter 1120 in a system for monitoring blood constituents. Figure 6 illustrates a general flow diagram for the steps of accounting for the imprecision in the filter to obtain the characteristics of a medium under test. Figure 20 illustrates a general functional diagram of the process of accounting for filter imprecision through signal processing. As depicted in Figure 19, the start of processing is represented in a begin block 1300. First, housekeeping and self-testing procedures are performed, as represented in an activity block 1305. Briefly, housekeeping and self testing involves boot operations and conventional initialization a self testing. For example, the system first determines if there is a sufficient signal intensity to take an accurate reading. After housekeeping and self testing is completed, the fight source 1110 (Figures 16 and 21) is activated to transmit light 1115 through the filter 1120, as represented in an activity block 1310. Initially, the light source 1110 is activated while no test medium 1131 is interposed between the filter 1120 and the detector 1140. Thus, the light which is detected by a detector 1140 (Figure 16) represents a baseline light intensity ($I_o$) which can be used as a test to insure that a bulb which is too dim or too bright is not inserted as a replacement bulb for example. In one embodiment, a lens 1117 (Figure 21) can be provided between the light source and the filter 1120 to provide focused light 1115 on the filter 1120.

Once the initial baseline light intensity constant has been determined, the medium 1131 under test is inserted as indicated in an activity block 1312.

As indicated within an activity block 1315, the light which is incident upon the detector 1140 is converted to an electrical signal and this signal is amplified in a pre-amp (not shown), filtered with the band pass filter (not shown), and sampled by an analog-to-digital converter 1142. Since the filter 1120 is rotating (at approximately 78.125 revolutions per second in one actual embodiment, although other rotational rates could be advantageous as called for by the particular application), samples of the electrical signal output by the detector 1140 are indicative of the light intensity detected at various rotational positions of the filter 1120. In one advantageous embodiment, one complete rotation (i. e., 360°) of the filter 1120 corresponds to 512 digital samples. That is, 512 samples are taken within the period corresponding to one revolution of the filter 1120. Thus, for example, if the filter 1120 rotates at 78.125 revolutions per second, then 512 samples will be taken within approximately 1/78th of a second, so that the sampling rate of the analog-to-digital converter 1142 will be approximately 40,000 samples per second.

As described, the filter 1120 constructed in accordance with the present invention includes redundant regions within an entire revolution. Specifically, the filter 1120 is symmetrically layered so that the first half-revolution of the filter provides a mirror of the signal of the second half-revolution of the filter 1120. That is to say, as depicted in Figure 15, the filter is formed in a wedge shape so that the thickness in one direction is constant and the thickness in the perpendicular direction increases linearly. Thus, the second half-revolution of the filter 1120 is redundant. For this reason, digital samples taken for one-half of the revolution of the filter 1120 could be discarded so that in each rotation of the filter 1120 there are 256 samples used for purposes of digital signal processing rather than 512 samples in the embodiment described above. Alternatively, all 512 samples can be used for processing by averaging corresponding values. In yet an alternative embodiment, the redundant half of the filter may be used for filter and source calibration. Each of the 256 samples (if only half are used) represents a different portion of the filter 1120 having different optical transmission characteristics.

Advantageously, the filter 1120 is specially designed to include an opaque strip (i.e., the brass strip 1122). The digital signal processor 1145 detects when the opaque strip 1122 of the filter 1120 is interposed between the light 1115 and the detector 1140 by monitoring the intensity output from the detector 1140. This intensity is effectively zero when the light is blocked by the opaque strip 1122. Since the opaque strip 1122 blocks substantially all of the optical radiation transmitted from the source 1110, any signal output from the optical detector 1140 when the light is blocked (e.g., from ambient light, thermal effects, etc.), will be interpreted as electrical noise which is not due to either the spectral absorption characteristics of the medium under test 1131 or the spectral transmission characteristics of the filter 1120. Thus, the digital signal processor 1145 interprets the signal present at the output of the optical detector 1140 when the brass strip 1122 is interposed between the fight source 1110 and the optical detector 1140 as stochastic noise which is subsequently subtracted from all signals output from the optical detector 1140. In one embodiment, this is simply accomplished by subtracting the digital value corresponding to the detected noise level from each of the digital values corresponding to the detected signal samples obtained within the activity block 1315. Alternatively, a shutter mechanism could be interposed within the light path, or the lamp 1110 could be turned off momentarily to provide the same effect. In this manner, the electrical noise inherent within the system is removed so that those electrical signals due to the optical transmission characteristics of the filter 1120 (and the test medium 1130) are considered in the further processing steps.

Once the stochastic noise inherent within the system has been extracted, control passes from the activity block 1315 to an activity block 1323. Within the activity block 1323 the signal is divided by $I_o$ to normalize the signal. The normalized signal is subsequently processed within an activity block 1325 to construct a signal intensity matrix, or vector, from the sample values obtained within the activity block 315 (taking into consideration the subtraction of the electrical noise, and the signal normalization performed in the activity block 1323) Figure 21 illustrates a signal intensity matrix $I_{\phi m}$.

The signal intensity matrix 1000 (Figure 21) is a one column matrix (sometimes referred to as a vector) including 256 signal intensity values (e.g., one value for each sampled rotational position of the filter 1120 in the present embodiment). Thus, the signal intensity vector 1000 is obtained by direct measurement of the optical signal which passes through both the filter 1120 and the test medium 131 and is detected by the optical detector 1140. Of course, the values used to form the signal intensity vector 1000 are taken from the amplitude of the signals output from the detector 1140 after subtraction of the noise from each sample. Designating each rotational position of a filter 1120 which is sampled by the analog-to-digital converter 1170 by the symbol $\phi$, then $\phi_1$ will correspond to the first rotational position of the filter 1120, $\phi_2$ will correspond to the second rotational position of the filter 1120, to $\phi_{256}$, which corresponds to the last rotational position of the filter 1120 before $\phi_1$ is taken again. Using this notation, $I_{\phi 1}$ corresponds to the intensity of light detected by the optical detector 1140 when the filter 1120 is in the first rotational position $\phi_1$, $I_{\phi 2}$ corresponds to the intensity of light detected by the detector 1140 when the filter 1120 is in the second rotational position $\phi_2$, etc. Thus, the signal intensity matrix comprises a single column matrix having 256 digital values from $I_{\phi 1}$ to $I_{\phi 256}$, which correspond to the optical intensities detected at each of the rotational positions of the filter 1120. In one embodiment, the intensity values for several revolutions are averaged to form the signal intensity matrix.

Once the signal intensity vector has been obtained in activity block 1325 (Figure 19), hereinafter designated as $I(\phi)$, and the filter characteristics matrix, hereinafter designated as $F(\phi,\lambda)$, has been obtained as explained above and represented as a data input in a block 1333, the signal intensity matrix together with the filter characteristics matrix may be used to obtain a matrix indicative only of the optical absorption characteristics of the test medium 1131, as represented in activity blocks 1330, 1331. That is, since the overall optical absorption is known as measured within the signal intensity matrix, $I(\phi)$, and the optical transmission characteristics of the filter 1120 are known as represented by the filter characteristics matrix, $F(\phi,\lambda)$ the optical absorption of the detected light due to the characteristics of the test medium 1131 may be determined by removing the optical transmission characteristics due to the filter from the overall intensity vector $I(\phi)$ combined. This is accomplished by first taking the inverse transform of the filter matrix, as represented in the activity block 1331, and subsequently multiplying the signal intensity vector $I(\phi)$ by the inverse filter matrix, as represented in the activity block 1330.

If the transmission through the test medium 1131 is designated as $T(\lambda)$ wherein the transmission of light through the test medium 1131 is defined as a function of the wavelength, and the transmission of light through a selected rotational position (e.g., when $\phi = 0$, corresponding to 0°) of a filter 1120 is maintained as a function of wavelength and is designated by the function $F(\phi,\lambda)$, the combination, or convolution, of the optical absorption due to the test medium 1131 and the filter 1120 is designated over the same wavelengths by the function $I(\phi)$. To obtain $T(\lambda)$ from the intensity vector $I(\phi)$ and the filter transmission matrix $F(\phi,\lambda)$, the intensity vector $I(\phi)$ and the inverse $F^{-1}(\phi\lambda)$ are multiplied.

The functions $I(\phi)$ and $F(\phi,\lambda)$ may be represented by the signal intensity and filter characteristic matrices, respectively. Thus, since

$$I(\phi) = F(\phi,\lambda) \times T(\lambda) \tag{13}$$

and $I(\phi)$ represents a one-column matrix (vector) containing an intensity value for each rotational position value $\phi$, while $F(\phi,\lambda)$ represents a two dimensional matrix containing a filter transmission coefficient value for each value of $\phi$ and each value of $\lambda$ (Figure 17D), then the function $T(\lambda)$, representative of optical transmission through the test medium 1131. may be represented as a one column matrix having values for each of the various wavelength values, $\lambda$.

In accordance with one embodiment of the present invention, 16 wavelengths are selected over the range of 850 nanometers to 1,400 nanometers for purposes of characterizing the spectral characteristics of the test medium 1131 as well as the filter 1120.

The matrix form of equation (1) above is shown below:

$$
\begin{matrix} I(\phi) & & F(\phi,\lambda) & & T(\lambda) \end{matrix}
$$

$$
\begin{bmatrix} I_{\phi 1} \\ I_{\phi 2} \\ \vdots \\ I_{\phi m} \end{bmatrix} = \begin{bmatrix} f_{\phi 1 \lambda 1} & f_{\phi 1 \lambda 2} & \cdots & f_{\phi 1 \lambda n} \\ f_{\phi 2 \lambda 1} & \ddots & \cdots & \vdots \\ \vdots & \vdots & \cdots & \vdots \\ f_{\phi m \lambda 1} & \cdots & \cdots & f_{\phi m \lambda n} \end{bmatrix} \cdot \begin{bmatrix} t_{\lambda 1} \\ t_{\lambda 2} \\ \vdots \\ t_{\lambda n} \end{bmatrix} \quad (14)
$$

As shown in Equation (2), the signal intensity matrix I(ϕ) is equal to the product of the two dimensional filter characteristic matrix, F(ϕ,λ), and the single column test medium matrix T(λ). In this equation, two of the matrices are given (i.e., I(ϕ) and F(ϕ,λ)). Thus, the third matrix, T(λ), which represents the optical transmission characteristics of the test medium 1131 for the 16 selected wavelengths between 850 nanometers and 1,400 nanometers, may be obtained by simply multiplying the inverse of the filter characteristic matrix, designated as F $^1$(ϕ,λ), by the signal intensity matrix, I(ϕ), using conventional matrix inversion and multiplication techniques, as shown below.

$$
\begin{matrix} T(\lambda) & & F^{-1}(\phi,\lambda) & & I(\phi) \end{matrix}
$$

$$
\begin{bmatrix} t_{\lambda 1} \\ t_{\lambda 2} \\ \vdots \\ t_{\lambda N} \end{bmatrix} = \begin{bmatrix} f_{\phi 1 \lambda 1} & f_{\phi 2 \lambda 2} & f_{\phi 1 \lambda m} \\ f_{\phi 2 \lambda 1} & \ddots & \vdots \\ \vdots & \vdots & \vdots \\ f_{\phi m \lambda 1} & \cdots & f_{\phi m \lambda n} \end{bmatrix}^{-1} \cdot \begin{bmatrix} I_{\phi 1} \\ I_{\phi 2} \\ \vdots \\ I_{\phi m} \end{bmatrix} \quad (15)
$$

Thus, as indicated in an activity block 1331, the inverse transform is taken of the filter characteristic matrix, F$^{-1}$(ϕ,λ), and then this inverse matrix is multiplied by the signal intensity matrix, I(ϕ), within the activity block 1330 to obtain the frequency response of the test medium 1131 as expressed by the test medium characteristic matrix, or transmission vector T(λ).

Figure 21 illustrates this operation in pictorial form. As shown in Figure 21, the light source 110 emits light which passes through the lens 1117 and the filter 1120 to provide filtered optical radiation 1125. The optical radiation 125 passes through the medium under test 1131 to provide an optical signal used to generate the signal intensity matrix 1000.

The signal intensity matrix 1000 is multiplied by the inverse of the filter characteristic matrix 1010 as indicated within a block 1005. As shown in Figure 21, the filter characteristic matrix 1010 is derived from an analysis of the filter 1120, as described above. The inverse transform of the filter characteristic matrix 1010 is multiplied by the signal intensity vector 1000 to obtain the optical frequency response matrix, or transmission vector, 1015.

Further processing depends on the desired analysis of the test medium.

APPLICATIONS INVOLVING BLOOD GLUCOSE MONITORING

As mentioned above, the filter could be used in monitoring blood glucose levels within a patient, such as a diabetic, without requiring the extraction of blood. This application is described briefly below.

Figure 3 schematically depicts the filter 1120 in operation as an optical filter within a blood glucose monitor. Optical radiation 1115 emitted from a light source 1110 is focused via a lens assembly 1117 (which may comprise fiber optics or the like) and passes through the filter 1120. The dichroic filter 1120 comprises an optically transmissive rotatable disk substrate which is layered with optical coatings having different thicknesses so as to modulate the broadband optical radiation 1115 through a spectrum from the near infrared (NIR) (e.g., 700 nm) to the infrared (IR) (e.g., 1,400 nm). The filter 1120 further includes the optically opaque strip 1122 which may, for example, comprise brass or some other metal which is deposited radially outward from the center of the filter disk 1120. The opaque strip provides a "0" location indicator and zero optical intensity, or electrical offset. The filter disk 1120 is driven in a circular motion by a smooth, disk drive motor in one preferred embodiment; however, a stepper motor could be used advantageously for its known phase condition. Filtered optical radiation 1125 passes from the filter 1120 through a fleshy medium, perfused with blood such as a finger tip 1130. In some applications, it may be desirable to provide a focusing lens, or other optical conduit, between the filter 1120 and the finger 1130. The light which passes through the finger 1130 is detected by a detector 1140. In general, the detection signal is conditioned and converted to digital form in the analog to digital conversion circuit 1142. The digital signal processor 1145 accepts the digital signals and accommodates for the imprecision in the dichroic filter.

In operation, when light 1115 is emitted from the broadband light source 1110 over a wavelength range of approximately 700 nanometers to 1,400 nanometers, (or 850-1700 nanometers in another embodiment where the upper and lower wavelengths have a ratio of approximately 2:1) this broadband light 1115 shines through the rotating dichroic filter 1120. It should be noted that the light 1115 is focused onto a portion of the filter 1120 by means of fiber optics, a lens assembly (e.g., the lens 1117), or the like. As the dichroic filter 1120 rotates, the broadband light 1115 is filtered through a portion of the dichroic filter 1120 producing the filtered optical radiation 1125. As indicated above, the dichroic filter 1120 is coated with optical layers of varying thickness so that different portions of the dichroic filter 1120 pass different wavelengths of light. Thus, as the filter 1120 rotates, the optical radiation 1125 output from the filter includes optical radiation of various wavelengths. In one embodiment, a fiber optic is used to couple the optical radiation 1125 emitted from a portion of the filter 1120 to the patient's finger 1130. It should be noted here, that since the optical characteristics of the filter 1120 can be carefully measured and the rotational speed of the dichroic filter 1120 is known, the time-varying pattern of optical radiation 1125 emitted from the filter 1120 to illuminate the finger 1130 is well defined, and therefore, may be used during signal processing to determine the amount of attenuation which is due to the optical filter 1120.

The optical radiation 1125 which is used to illuminate the finger 1130 passes through the finger 1130 to produce the detectable light 1135. As is well known in the art, some of the optical radiation 1125 passes unimpeded through the finger 1130, some of the optical radiation 1125 is reflected within the finger 1130 to produce scattering. The scattered radiation which is transmitted through the finger 1130, together with the light which passes unimpeded through the finger 1130, make up the light 1135. Some of the optical radiation 1125 is absorbed by constituents within the finger 1130.

The finger 1130 is known to include a fingernail, skin, bones, flesh, and blood. The blood itself primarily comprises water, oxyhemoglobin, hemoglobin, lipids, protein and glucose. Each of these constituents within the finger (e.g., nerves, muscle tissue, etc.) contribute to the absorption and scattering of the optical radiation 1125 through the finger 130. The absorption of optical radiation through a nonhomogeneous medium typically follows well defined laws in relation to the optical characteristics of each of the constituents taken separately. Approximations to these laws are expressed in the equations for Beer-Lambert's law, where low scattering applications most closely follow the Beer-Lambert equations. The light 1135 which passes through the finger 1130 is incident upon the optical detector 1140. The optical detector 1140 generates an electrical signal proportional to the overall intensity of the light 1135.

Although the light 1135 typically has different intensities at different wavelengths, the optical detector 1140 generates an electrical signal which is proportionate to the area contained under the spectral response curve of the light 1135 within the optical band detected by the detector 1140. That is, the optical detector 1140 receives light having different intensities at different wavelengths. The detected wavelengths are restricted over a band of approximately 850 nm to 1,700 nm due to the characteristics of the detector 1140, so that, if intensity is plotted as a function of wavelength to obtain a spectral response curve, the area under the spectral response curve will be indicative of the average optical radiation intensity incident upon the detector 1140. Thus, the electrical signal produced by the detector 1140 is proportional to the overall (i.e., average) intensity of the light 1135.

The filter 1120, constructed in accordance with the present invention, includes redundant regions within an entire revolution. Specifically, the filter 1120 is symmetrically layered so that the first half-revolution of the filter is substantially symmetrical to the signal of the second half-revolution of the filter 1120. That is to say, as depicted in Figure 15, the filter is formed in a wedge shape so that the thickness in one direction is constant and the thickness in the perpendicular direction increases linearly. Thus, the second half-revolution of the filter 1120 is redundant. For this reason, digital samples taken for one-half of the revolution of the filter 1120 could be discarded so that in each rotation of the filter 1120 there are 128 samples used for purposes of digital signal processing rather than 256 samples in one embodiment. Of course, it will be appreciated that some of the samples are lost due to the opaque strip. Alternatively, all 256 samples can be used for processing by averaging corresponding values. In yet an alternative embodiment, the redundant half of the filter may be used for filter and source calibration. Each of the 128 samples (if only half are used) represents a different portion of the filter 1120 having different optical transmission characteristics.

PRODUCTION SPECIFICATIONS FOR THE OPTICAL FILTER

In one advantageous embodiment for blood glucose measurement, the production specifications for the filter 1120 are as follows:

| | |
|---|---|
| SIZE: | 20 mm wide x 20 mm wavelength span, linear multilayer coating |
| SUBSTRATE: | 25 mm 0D glass disc with 7.5 mm shaft hole in center |
| WAVELENGTH PASSED: | 700-1400 nanometers |
| 1/2 BANDWIDTH: | 50 to 200 nanometers, bands may repeat |
| BLOCKING: | none |
| ENVIRONMENT: | Survive condensing humidity, 0.70 c |

The pass band edges are produced so as to differentiate a 20 nanometer band edge.

The pass band may repeat within the window at as little as 400 $cm^{-1}$ spacing, or 17-18 periods within the window. The pass band center transmission should approach 100%, and the region between pass bands should approach 100% reflection.

Blocking requirements outside of the window are not critical. They may be limited by band-edge materials such as RG660, RG700, or semiconductors, or O-H bands typically found in glass below 7100 $cm^{-1}$.

Only the ability to resolve wave number bands near 200 $cm^{-1}$ with one or more band edges should 'imit the cost.

CHARACTERISTICS FOR PRESENT EMBODIMENT

Preferably, the filter will not have a window narrower than 8,000 to 11,000 $cm^{-1}$ or about 910 to 1,250 nm. The bandwidth is advantageously wider than 200 $cm^{-1}$, and the band edge is advantageously narrower than 200 $cm^{-1}$. The transmission maximum of the primary band is advantageously above 80%, and the transmission minimum is advantageously below 20%. Any other bands should be repeatable, unit to unit; but if they are not, a calibration ROM could be used in accordance with the DSP to perform initial calibration of individual filters.

MECHANICAL BOUNDARIES AND CHARACTERISTICS FOR THE PRESENT EMBODIMENT

The linear filter is advantageously rotated about its center at less than 4,800 RPM for portable in vivo applications (although near 48,000 RPM might be suitable in certain industrial applications), with an aperture centered at a radius of minimum 9 mm to maximum 45 mm, with a clear aperture diameter of 1 mm to 3 mm and a numerical aperture of .12 to .40. The light path passes through a small circular portion traveling along an annular region of the rotating filter, causing a sinusoidal scan of the wavelengths, although they are deposited linearly.

For dynamic balance and low turbulence, the linear filter is deposited on a circular substrate. Since the center is not used optically, a standard diameter shaft mounting hole is preferred; most of the present hardware in the invention use either 0.5000-.000, +.0005" diameter, or 7.5 -0.0+.1 mm. For a small filter, e.g., 20 mm diameter, bonding to the uncoated side would be considered. Note that the filter mount does not have spokes or other structural interruption of the optical path.

Although the preferred embodiment of the present invention has been described and illustrated above, those skilled in the art will appreciate that various changes and modifications to the present invention do not depart from the scope of the invention. For example, the principles and method of the present invention could be used to detect trace elements within the bloodstream (e.g., for drug testing, etc.). Accordingly, the scope of the present invention is limited only by the scope of the following appended claims.

**Claims**

1. A system (100) configured to non-invasively monitor a blood constituent in tissue of a living subject, said system (100) having a light source (301), which is adapted to emit a plurality of wavelengths and positioned to transmit radiation through a field of view area of said tissue, and an optical detector (320) positioned to detect the transmitted radiation after propagating through said tissue, said optical detector (320) configured to generate an output signal indicative of the intensity of said radiation after attenuation through said fleshy medium, said system further comprising:

   an active pulse device (150) adapted to induce a periodic change in the volume of blood in the fleshy medium with an inducement level selected to cause no more than minimal perturbation to the optical properties in the tissue in the field of view area, wherein the inducement level is sufficient to cause a modulation in the detected signal between about 1% and about 10%; and
   a signal processor (334) coupled to said detector (320) and responsive to said output signal to analyze said output signal to extract portions of said signal due to optical characteristics of said blood to determine the concentration of said constituent within said subject's bloodstream.

2. The system of Claim 1, wherein the active pulse device (150) is adapted to cause a periodic change in the volume of blood in the fleshy medium independent of the natural flow of blood in said fleshy medium.

3. The system of Claim 1, wherein the active pulse device (150) is adapted to cause a periodic change in the volume of blood in the fleshy medium in conjunction with the natural flow of blood in said fleshy medium.

4. The system of Claim 1, wherein said constituent is blood glucose.

5. The system of Claim 1, further comprising a receptacle which receives said fleshy medium, said receptacle further having an inflatable bladder (516).

6. The system of Claim 1, further comprising a receptacle which receives said fleshy medium, said receptacle further comprising a temperature variation element, said temperature variation element cyclicly varying the temperature of said fleshy medium in order to induce a change in the flow of blood in said fleshy medium.

7. The system of Claim 1, wherein said active pulse device (150) is adapted to induce a periodic change in the volume of blood in the fleshy medium with an inducement level sufficient to cause a modulation in the detected signal of about 1% to about 5%.

**Patentansprüche**

1. System (100), das dazu eingerichtet ist, nicht-invasiv einen Blutbestandteil im Gewebe eines Lebewesens zu überwachen, wobei das System (100) eine Lichtquelle (301), die angepaßt ist, mehrere Wellenlängen zu emittieren und angeordnet ist, um Strahlung durch ein Gebiet eines Beobachtungsbereichs des Gewebes zu senden, und einen optischen Detektor (320) aufweist, der angeordnet ist, um die gesendete Strahlung nach der Ausbreitung durch das Gewebe zu detektieren, wobei der optische Detektor (320) dazu eingerichtet ist, ein Ausgangssignal zu erzeugen, das für die Intensität der Strahlung nach einer Schwächung durch das fleischige Medium kennzeichnend ist, wobei das System ferner aufweist:

   eine aktive Impulsvorrichtung (150), die angepaßt ist, eine periodische Änderung im Blutvolumen im fleischigen Medium mit einem Induzierungspegel zu induzieren, der so ausgewählt ist, daß er nicht mehr als eine minimale Störung der optischen Eigenschaften im Gewebe in dem Gebiet des Beobachtungsbereichs verursacht, wobei der Induzierungspegel ausreichend ist, eine Modulation im detektierten Signal zwischen etwa 1% und etwa 10% zu bewirken; und
   einen Signalprozessor (334), der mit dem Detektor (320) gekoppelt ist und auf das Ausgangssignal reagiert, um das Ausgangssignal zu analysieren, um Anteile des Signals zu extrahieren, die auf die optischen Eigenschaften des Bluts zurückzuführen sind, um die Konzentration des Bestandteils im Blutstrom des Lebewesens zu bestimmen.

2. System nach Anspruch 1, wobei die aktive Impulsvorrichtung (150) angepaßt ist, eine periodische Änderung im Blutvolumen im fleischigen Medium zu bewirken, die unabhängig vom natürlichen Blutstrom im fleischigen Medium ist.

**3.** System nach Anspruch 1, wobei die aktive Impulsvorrichtung (150) angepaßt ist, eine periodische Änderung im Blutvolumen im fleischigen Medium in Verbindung mit dem natürlichen Blutstrom im fleischigen Medium zu bewirken.

**4.** System nach Anspruch 1, wobei der Bestandteil Blutzucker ist.

**5.** System nach Anspruch 1, das ferner ein Behältnis aufweist, das das fleischige Medium aufnimmt, wobei das Behältnis ferner eine aufblasbare Blase (516) aufweist.

**6.** System nach Anspruch 1, das ferner ein Behältnis aufweist, das das fleischige Medium aufnimmt, wobei das Behältnis ferner ein Temperaturvariationselement aufweist, wobei das Temperaturvariationselement zyklisch die Temperatur des fleischigen Mediums variiert, um eine Änderung im Blutstrom im fleischigen Medium zu induzieren.

**7.** System nach Anspruch 1, wobei die aktive Impulsvorrichtung (150) angepaßt ist, eine periodische Änderung im Blutvolumen im fleischigen Medium mit einem Induzierungspegel zu induzieren, der ausreicht, um eine Modulation im detektierten Signal von etwa 1% bis etwa 5% zu verursachen.

## Revendications

**1.** Système (100) configuré pour contrôler de manière non destructrice un constituant du sang dans le tissu d'un être vivant, le système (100) possédant une source de lumière (301) qui est conçue pour émettre une pluralité de longueurs d'onde et est positionnée pour émettre un rayonnement dans une zone d'angle de champ dudit tissu, et un détecteur optique (320) qui est positionné pour détecter le rayonnement émis après propagation dans ledit tissu, ledit détecteur optique (320) étant configuré pour produire un signal de sortie indicatif de l'intensité du rayonnement après atténuation dans ledit milieu charnu, ledit système comprenant en outre :

un dispositif à impulsions actives (150) conçu pour induire une variation périodique du volume du sang dans le milieu charnu avec un effet d'entraînement d'un niveau choisi pour n'amener rien de plus qu'une perturbation minimale dans les propriétés optiques du tissu se trouvant dans la zone d'angle de champ, où l'effet d'entraînement est d'un niveau suffisant pour amener une modulation du signal détecté comprise entre environ 1 % et environ 10 % ; et
un dispositif (334) de traitement de signaux couplé audit détecteur (320) et réagissant audit signal de sortie en analysant ledit signal de sortie pour extraire des parties dudit signal qui sont dues aux caractères optiques dudit sang et, ainsi, déterminer la concentration du sang dudit sujet en ledit constituant.

**2.** Système selon la revendication 1, où le dispositif (150) à impulsions actives est conçu pour amener une variation périodique du volume du sang dans le milieu charnu qui est indépendante de la circulation naturelle dans ledit milieu charnu.

**3.** Système selon la revendication 1, où le dispositif (150) à impulsions actives est conçu pour amener une variation périodique du volume du sang dans le milieu charnu en liaison avec la circulation naturelle du sang dans ledit milieu charnu.

**4.** Système selon la revendication 1, où ledit constituant est le glucose sanguin.

**5.** Système selon la revendication 1, comprenant en outre un réceptacle qui reçoit ledit milieu charnu, ledit réceptacle possédant en outre une vessie gonflable (516).

**6.** Système selon la revendication 1, comprenant en outre un réceptacle qui reçoit ledit milieu charnu, ledit réceptacle comprenant en outre un élément de variation de la température, ledit élément de variation de la température faisant varier cycliquement la température dudit milieu charnu afin d'induire une modification de la circulation du sang dans ledit milieu charnu.

**7.** Système selon la revendication 1, où ledit dispositif (150) à impulsions actives est conçu pour induire une variation périodique du volume du sang dans le milieu charnu avec un effet d'entraînement d'un niveau suffisant pour provoquer une modulation du signal détecté entre environ 1 % et environ 5 %.

*FIG. 1*

FIG. 2

FIG. 2A

FIG. 2B

RADIATED POWER

FIG. 2C

t

AA

BB

FIG. 3

COMPOSITE SIGNAL

300

330

GAIN SET BY DSP

342 PRE-AMPLIFIER

344 HIGH PASS FILTER

346 AMPLIFIER

343 348 PROGRAM GAIN AMPLIFIER

350 LOW PASS FILTER

332

352 356 16 BIT NOISE SHAPING A/D CONVERTER

150KHZ DATA (TO DSP)

347 354 16 BIT A/D CONVERTER

(TO DSP)

*FIG. 4*

*FIG. 5*

1st λ +AMBIENT          2nd λ +AMBIENT          3rd λ +AMBIENT

AMBIENT                 AMBIENT                 AMBIENT

MODULATED SIGNAL (80 SAMPLES)

*400*

MODULATED
DATA AT
50KHz SAMPLES → dMUX → *422* 1st λ → *430* SUM UP 4 SAMPLES *432* → + *438* *442* 1/4 → 1st λ AT 625 Hz
AMBIENT → SUM UP 4 SAMPLES → −
*426* *424*
*421*
SELECT        *428*
2nd λ → *434* SUM UP 4 SAMPLES → + → 1/4 → 2nd λ AT 625 Hz
AMBIENT → SUM UP 4 SAMPLES → −  *440* *444*
*436*

*FIG. 6*

FIRST CHANNEL (625Hz) → FIR LOWPASS FILTER (519 SAMPLES) `450` → DECIMATION BY 10 `454` → OUTPUT BUFFER (570 SAMPLES) `458` → FIRST SNAPSHOT (62.5 Hz)

SECOND CHANNEL (625Hz) → FIR LOWPASS FILTER (519 SAMPLES) `452` → DECIMATION BY 10 `456` → OUTPUT BUFFER (570 SAMPLES) `460` → SECOND SNAPSHOT (62.5 Hz)

`402`

FIG. 7

EP 0 831 738 B1

FIG. 8

FIG. 9

FIG. 10

FIG. 11

*FIG. 12a*

*FIG. 12*

FIG 13

*1150*

*1156*

*1154*

*1152*

FIG. 14

(PRIOR ART)

*FIG. 15*

FIG. 16

λ = 850nm

*FIG. 17A*

TRANSMISSION

100%

50%

0   32   64   96   128   160   192   224   256

DISC ROTATION

λ = 1150nm

*FIG. 17B*

TRANSMISSION

100%

50%

0   32   64   96   128   160   192   224   256

DISC ROTATION

λ = 1350nm

*FIG. 17C*

TRANSMISSION

100%

50%

0   32   64   96   128   160   192   224   256

DISC ROTATION

$$\begin{bmatrix} f_{\phi_1\lambda_1} & f_{\phi_1\lambda_2} & \cdots & f_{\phi_1\lambda_n} \\ f_{\phi_2\lambda_1} & f_{\phi_2\lambda_2} & & \\ \vdots & & \ddots & \\ f_{\phi_m\lambda_1} & \cdots & & f_{\phi_m\lambda_n} \end{bmatrix}$$

*FIG. 17D*

FIG. 18

FIG. 19

FIG. 20

FIG 21